# EUROPÄISCHE PATENTSCHRIFT

(11) **EP 3 606 597 B1**
(45) Veröffentlichungstag und Bekanntmachung des Hinweises auf die Patenterteilung: **31.07.2024**
(21) Anmeldenummer: 18721276.6
(22) Anmeldetag: 29.03.2018
(51) Int. Cl.: A61M 27/00

(54) **FLUSSREDUKTOR**
FLOW REDUCER
RÉDUCTEUR DE DÉBIT

(30) Priorität: 06.04.2017 DE 102017003366; 28.07.2017 DE 102017007093; 06.02.2018 DE 102018000941; 21.02.2018 DE 102018001392
(43) Veröffentlichungstag der Anmeldung: 12.02.2020
(73) Patentinhaber: Christoph Miethke GmbH & Co. KG, 14469 Potsdam (DE)
(72) Erfinder: MIETHKE, Christoph, 14469 Potsdam (DE)
(74) Vertreter: Greiche, Albert
(86) Internationale Anmeldenummer: PCT/EP2018/000135
(87) Internationale Veröffentlichungsnummer: WO 2018/184717

(56) Entgegenhaltungen:
- EP-A1- 2 359 886
- EP-A2- 2 777 751
- WO-A1-91/17779
- WO-A2-2014/144703
- JP-A- 2006 020 905
- US-A- 3 566 875
- US-A1- 2011 275 976
- US-A1- 2012 226 215
- US-A1- 2013 085 441

## Beschreibung

Die Erfindung betrifft ein Hydrocephalusventil zum Abfluss von Fluid aus Ventrikelsystemen von Patienten, dass mindestens ein Gehäuse mit Gehäuseinnenraum, mindestens einen ersten Durchlass zum Einlassen und/oder Auslassen umfasst, mit mindestens einem Körper, welcher im Gehäuseinnenraum angeordnet ist, wobei der Körper mindestens in einer Richtung bewegbar ausgebildet ist und das mindestens eine Einstelleinheit aufweist.

Hydrozephalus Patienten haben folgendes medizinisches Problem:
Das Gehirn ist im Schädel umgeben von einer besonderen Flüssigkeit, dem Liquor. Dieser Liquor wird ständig produziert und im gleichen Maße resorbiert. Bei der Erkrankung des Hydrozephalus, auch Wasserkopf genannt, ist dieses Gleichgewicht gestört. Da der Schädel ein geschlossenes Gefäß darstellt, kommt es zu einer Vergrößerung, wenn mehr Liquor produziert als resorbiert wird. Durch die Vergrößerung können beim Säugling die Schädelnähte nicht zusammenwachsen, beim Erwachsenen steigt der Schädelinnendruck. Es gibt also ein Alters- und Kinderhydrozephalus.

Hydrozephalus lässt sich in seinem Formen in Hydrocephalus internus, Hydrocephalus externus, Hydrocephalus externus et internus, Normaldruckhydrocephalus und Hydrocephalus e vacuo unterscheiden.

Die Behandlung eines Hydrozephalus erfolgte ursprünglich durch die bloße Ableitung des Liquors. Dies geschah durch die bloße Schlauchverbindung zwischen dem Schädel und einem großen venösen Blutgefäß oder durch eine entsprechende Verbindung des Schädels über einen Schlauch mit dem Bauchraum. Bald erkannte man jedoch, dass der Druck im Schädel einen bestimmten physiologischen Wert besitzen muss, wenn nicht wieder andere Komplikationen auftreten sollen.

Moderne Therapien des Hydrocephalus nutzen eine implantierbare Drainage, eine künstliche Verbindung zwischen den Hirnkammern im Kopf und einem Ableitungskompartiment, heute meistens dem Bauchraum zur Einstellung eines bestimmen physiologischen Werts.

Es sind verschiedene Drainagen bekannt mit denen der Druck im Schädel eines Patienten therapiert werden kann. Die Drainagen sollen sich bei einem bestimmten kritischen Druck öffnen und den Abfluss von Liquor -auch Cerebrospinalflüssigkeit genannt- freigeben, so dass eine Bildung eines Überdrucks im Schädel verhindert wird. Üblicherweise werden diese Drainagen zum Schutz vor einem Überdruck an Cerebrospinalflüssigkeit (CSF) als sogenannte Shunts oder Ableitungen bezeichnet.

Kern der implantierbaren Drainage ist ein implantierbares Ventil, mit dem die Drainage gesteuert wird. Dieses Ventil wird als Hydrocephalusventil bezeichnet. Die Hydrocephalusventile werden regelmäßig dicht unter der Haut eingepflanzt. Derartige Drainagen werden im Allgemeinen im Bereich des Kopfes unter der Haut implantiert.

Eine mögliche Definition des Begriffs Shunts ist nach Miethke: *jede künstliche hydraulische Verbindung zwischen einem ersten Körperteil, das Cerebrospinalflüssigkeit enthält und einem zweiten Körperteil, welches selbige aufnehmen* kann, vgl. The Cerebrospinal Fluid Shunts, Seite 130/131, (Quelle 1). Zum Thema Hydrozephalus sind weitere Quellen das Buch Normal Pressure Hydrozephalus, Fritsch et al., 2014 (Quelle 2) sowie die Normen EN ISO 7197 (Quelle 4) und EN ISO 1463 (Quelle 5).

Alle Quellen enthalten u.a. Fachbegriffe und Definitionen zum Thema Hydrocephalus. Ferner enthalten sie bekannte Wirkprinzipien und deren Gruppierung.

Miethke schlägt in Quelle 1 eine zweifache Gruppierung vor, vgl. dazu Tabelle 1. In einer ersten Untergruppierung unterteilt er Ventile nach ihrem Wirkprinzipien in Differenz-Druckventile und Hydrostatische Ventile. In einer zweiten Untergruppierung differenziert er Ventile nach klinischen Funktionen, in feste, also nicht einstellbare und einstellbare Ventiltypen.

**Tabelle 1: Wirkprinzipen von Shunts, Quelle 1, Seite 117**

| Ventil | Fest | Einstellbar |
|---|---|---|
| Differenz-Druckventile | | |
| Silikonschlitzventile | X | |
| Membranventile | X | |
| Kugelkonusventile | X | X |

| Hydrostatische -Ventilprinzipien | | |
|---|---|---|
| Anti Siphon Device Prinzipien | X | |
| Fluss reduzierende Prinzipien | X | |
| Gravitationsbasierte Prinzipien | X | X |

Die Ventile der Gruppe des hydrostatischen Ventilprinzips sind nach Miethke als Ventile oder Ventilkomponenten bezeichnet, deren Konstruktionsziel in einem Verhindern einer Überdrainage begründet (Quelle 1, *Seite 67).* Ziel der Ventile dieser Gruppe ist dabei eine in Richtung einer Ventilöffnung wirkende Kraft eines hydrostatischen Drucks auszugleichen (sogenanntes Counterbalance).

Ventile mit hydrostatischem Wirkprinzip können in drei Ventilarten differenziert werden. Deren Bezeichnungen sind Anti-Siphon-, flusskontrollierte- und gravitationskontrollierte Geräte. Allen drei Ventilarten ist ein Differenzdruck gemein. Dieser berechnet sich aus der Differenz zwischen dem Druck hinter dem Ventil abzüglich des Drucks vor dem Ventil (Δp = p_{hinter dem Ventil} - p_{vor dem Ventil}). Die Druckdifferenz, welche einen Volumenstrom durch das Ventil freigibt, wird als Öffnungsdruck des Ventils definiert.

Anti-Siphon Geräte passen ihren Öffnungsdruck an die Höhe einer im Ventil angreifenden Saugkraft an. Gravitationskontrollierte Geräte passen den Öffnungsdruck an ihre Inklination im Gravitationsfeld der Erde an. Flusskontrollierte Geräte hingegen, passen den sie passierenden Volumenstrom an die Druckdifferenz an.

Ähnliche Bezeichnungen für Volumenstrom anpassende Ventile im Stand der Technik sind flussratenabhängige-, flussregulierende- oder flussreduzierende Ventile oder Geräte. Dabei ist der Begriff *Fluss* im Allgemeinen dem Begriff Volumenstrom, Volumen pro Zeit gleichzusetzen.

Jedes Hydrocephalusventil ist durch eine Kennlinie gekennzeichnet. Dr. med. Alfred Aschoff beschreibt Kennlinien in In-Vitro-Testung von Hydrozephalus Ventilen, 1994, Seite 32 (Quelle 7). Er nimmt ihre Diskussion darin auf, weil Shuntventile Strömungsregler mit unidirektionaler Richtungsbevorzugung sind. Sie zeichnen sich nach Aschoff dadurch aus, dass sie erstens durch eine unidirektionale Wirkung, zweitens durch einen Öffnungs- und Schließcharakteristik und drittens durch eine spezifische Druck-Fluss-Kennlinie charakterisiert sind. Die Druck-Fluss-Kennlinie ist üblicherweise nichtlinearer. Ihr Verlauf hänge nach Aschoff vom Hydrocephalusventil selber ab, so dass eine Hydrocephalusventil nur durch eine Angabe der vollständigen Kennlinie beschrieben werden kann.

Nicht einstellbare Hydrocephalusventile sind durch eine Ventilkennlinie gekennzeichnet, hingegen einstellbare durch mehrere Ventilkennlinien.

Bei den nicht einstellbaren Hydrocephalusventilen ist evident, dass Ventile der Gruppe des hydrostatischen Ventilprinzips in Abhängigkeit vom Liquordruck einen bestimmten Volumenstrom, eine Durchströmung zeigen. Wenn zu jedem Liquordruck ein zugehöriger Volumenstrom in einer Grafik aufgezeichnet wird, ergibt sich eine Ventilkennlinie.

Bei den einstellbaren Hydrocephalusventilen konfiguriert jede Einstellung das Ventil. Jede Konfiguration ergibt eine andere Ventilkennlinie. Im Folgenden sind einige bedeutsame Hydrocephalusventile beschrieben:

JP2006020905 (Kaneka Medix Corp) betrifft ein implantierbares Hydrocephalussystem Einheit, die in der Lage ist, auf einfache Weise einen Aktionseinstellungszustand einer subkutan implantierten Vorrichtung subkutan zu erfassen und dementsprechend die subkutan implantierte Vorrichtung sicher im richtigen Aktionszustand zu halten.

Die US 8870809 B2 (Christoph Miethke GmbH & Co KG) betrifft ein implantierbares Hydrocephalussystem zur Behandlung von Hydrocephalus Patienten mit Medikamenten. Die Druckschrift zeigt ein implantierbares Hydrocephalussystem, mit dem auch Medikamente in den Patienten verbracht werden können. Dazu sind die Medikamente in einen Hohlraum, eine Kavität des Hydrocephalussystems abzugeben, so dass sie von dort durch einen Ventrikelkatheter in die Gehirnventrikel hydraulisch verbringbar sind. Nach der Lehre ist dazu ein System notwendig, dass in einem Zustand medikamentöse Flüssigkeiten aufnimmt, und diese in einem anderen Zustand in Richtung der Gehirnventrikel verbringt. Das System setzt damit ein Ventil voraus und umfasst daher ein Ventil Arrangement mit einer Ventilklappe in einem Gehäuse mit einem Einlass und einem Auslass. Das Ventil Arrangement im Ventil öffnet oder schließt den Einlass des Hydrocephalussystems in Abhängigkeit des medikamentösen Flüssigkeitsdrucks in der Kavität.

Die DE 38 35 788 A1 lehrt gemäß EP 1 523 635 B1 Absatz [0003) (Aesculap AG) ein schnell, schaltendes Kugelventil. In einem Zustand wird medikamentöse Flüssigkeit aufgenommen und in einem anderen Zustand in Richtung der Gehirn abgegeben Im geschlossenen Zustand des Ventils wird die Kugel gegen die Durchlassöffnung gepresst. Zur Freigabe der Durchlassöffnung stößt der Betätigungsmechanismus die Kugel seitlich von der Durchlassöffnung weg. Hierzu gibt ein Betätigungselement des Betätigungsmechanismus einen seitlichen Stoß auf die Kugel, die sich daraufhin von der Durchlassöffnung bzw. dem Ventilsitz der Durchlassöffnung löst. Als Betätigungsmechanismus zum Verschieben der Kugel wird dabei ein impulsgetriebener Elektromagnet eingesetzt, der nach einer Betätigung durch eine Federkraft wieder in die Ausgangsposition zurückgezogen wird.

Die EP 1 523 635 B1 (Aesculap AG) bietet eine Lösung an, um ein Ventil bereitzustellen, dass Betätigungswege im Millimeterbereich ermöglicht. Dem Prinzip nach kombiniert der Vorschlag einen Grundkörper mit einer Durchlassöffnung und zwei drahtförmigen Elementen, insbesondere SMA- Drähten (ShapeMemory-Alloy) Diese verkürzen sich wechselseitig in Abhängigkeit einer Temperaturveränderung. In einer besonders vorteilhaften Ausgestaltung resultiert ein Ventil mit binärer Öffnungscharakteristik. Phenomenologisch resultiert eine Funktion eines Schalters aus einer Positionsmanipulation eines Körpers vor einer Durchlassöffnung.

US 2015 0182 734 A1 (Christoph Miethke GmbH & Co. KG) offenbart ein einstellbares Hydrocephalusventil, programmierbarer Gravitationsassistent, zur Druckeinstellung im Cranium eines Hydrocephalus-Patienten. Mittels einer Membran wird dazu eine Bremse gelöst, um einen Rotor freizugeben, so dass dieser frei um eine Achse rotiert werden kann. Die Membran meldet durch ein akustisches Signal, einem Klicken die Freigabe oder Blockade des Rotors an einen Nutzer. Weil in ihm Magnete verbaut sind, ist der Rotor mittels eines ebenfalls magnetischen Werkzeuges um seine Achse rotierbar. Die Rotation wird ausgenutzt um eine Ventilcharakteristik einzustellen. Das Ventil hat sich bewährt.

Die US 4676772 (Cordis-Cooperation) lehrte schon 1985 ein System zur Druckkontrolle von Cerebrospinalflüssigkeit. Dieses umfasst ein implantierbares Druckentlastungsventil für Fluide, welches ein Gehäuse und eine Einstelleinheit aufweist, um den Öffnungsdruck des Druckentlastungsventils einzustellen. In Abhängigkeit eines am Druckentlastungsventil anliegenden Drucks wird dabei eine Membran ausgelenkt, so dass ein Durchlass zwischen einem in der Membran eingelassenen Dichtring und einer Kugel geöffnet wird. Die Kugel ist dafür in einem Topf gelagert, in dessen Mantelfläche ein Gewinde geschnitten ist. Mittels des Gewindes ist der Topf in einen Deckel hinein- oder herausschraubbar, so dass ein Druck zwischen der Kugel und dem Dichtring einstellbar ist. Die Lage des Topfes, also die Anzahl eingeschraubter Gewindegänge ist im Druckentlastungsventil, kann über eine magnetische Brücke auf eine Anzeigevorrichtung wiedergegeben werden.

Die US 4676772 lehrt zusammenfassend eine Einstellung eines Ventilöffnungsdruckes, nachteiliger Weise hingegen nicht die Einstellung eines definierten Volumenstroms. Zudem weist die beschriebene Technik den Nachteil auf, dass ein Einstellen eines Ventilöffnungsdruckes durch Einschrauben eines Topfes eine plastische Verformung der Membran nach sich ziehen kann. Diese tritt auf, wenn durch ein zu starkes Einschrauben des Topfes über die Kugel eine Kraft auf die Membran aufgebracht wird, die jenseits der Elastizitätsgrenze der Membran ist. Ein präzises Einstellen eines Ventilöffnungsdrucks setzt ein präzises Positionieren des Topfes im Deckel voraus. Der Topf wird im Deckel über eine magnetische Brücke verdreht, die einer Handbewegung eines Anwenders entspricht. Der Anwender erhält allerdings kein Feedback über die Reibung oder die relative Position zwischen Topf und Deckel. Somit wird der Topf im Deckel auf Grund eines Über- oder Unterverdrehens durch den Anwender nicht präzise positioniert, so dass der Ventilöffnungsdruck nicht präzise einstellbar ist.

Das sogenannte Orbis-Sigma Ventil wurde von Sainte-Rose, Hooven und Hirsch in: A new approach in the treatment of hydrocephalus, Neurosrg, 1987, 66(2), 213-26, vorgestellt. Das Orbis Sigma Ventil umfasst eine Saphir Membran mit einer Bohrung sowie einen durch diese Bohrung durchstechenden Stift bzw. Pin. Dabei weist der Stift in seinem Querschnitt einen Hinterschnitt in Richtung seines membranzugewandten Endes auf. Die Membran ist entlang ihres Umfangs in einem Gehäuse in einem Strömungskanal gelagert. Der Stift ist an seinem membranabgewandten Ende im gleichen Gehäuse und gleichem Strömungskanal gelagert. Liegt ein Differenzdruck über der Membran an, weicht diese aus, in dem sie sich mit dem Druckgradienten wölbt. Die Stärke der Wölbung und die Form des Hinterschnittes im Stift definieren so dann einen Durchlass. Seine Größe variiert mit dem Verlauf des Hinterschnitts. Somit stellt das Orbis Sigma Ventil die Größe eines Durchlasses kontinuierlich entlang eines über einer Membran anliegenden Differenzdruckes im Zusammenspiel mit einem Verlauf eines Hinterschnittes ein.

Der Nachteil des Orbis-Sigma Ventils ist dessen Abhängigkeit vom Differenzdruck. Darüber hinaus kann der Verlauf des Hinterschnittes nicht für alle Patienten als konstant unterstellt werden. Vielmehr ist er auf die jeweilige Ausprägung des Hydrocephalus eines Patienten abzustimmen.

In der EP 0873761 B1 (DePuy) wird eine Vorrichtung zur Begrenzung einer Flüssigkeitsströmung beschrieben. Die Vorrichtung zeigt das Prinzip eines sogenannten *SiphonGuards*^{®}*.* Sie lehrte 1998 eine Technik, um einen Fluss eines Fluids von einem ersten Bereich eines Patienten zu einem zweiten Bereich zu begrenzen. Dazu umfasst die Vorrichtung einen Einlass, um das Fluid von einem ersten Bereich aufzunehmen und einen Auslass, um das Fluid in einen zweiten Bereich zu führen. Ferner umfasst die Vorrichtung einen primären Flussweg und einen sekundären Flussweg, die beide in Fluidkommunikation mit dem Einlass und dem Auslass stehen. Ein Detektor in der Vorrichtung kann die Flussrate, den Volumenstrom des Fluides detektieren, so dass in Abhängigkeit seiner Stärke über ein Führen desselben entlang des primären oder sekundären Flusswegs entschieden werden kann. Der Detektor führt dabei die Entscheidung herbei, in dem er eine aktuelle Flussrate mit einem Schwellwert vergleicht. Der Detektor führt das Fluid von dem Einlass zu dem Auslass entlang des primären Flusswegs, wenn die fluide Flussrate geringer als ein vorgegebene Schwellwert ist. Der Detektor führt umgekehrt das Fluid vom Einlass zum Auslass entlang des sekundären Fließweges wenn die Flussrate größer ist als ein vorgegebener Schwellwert. Der Detektor setzt sich dabei zusammen aus vier Bauteilen, einem Kugelsitz, eine Kugel, einer Blattfeder und einer Spiralfeder. Die Blattfeder drückt die Kugel aus dem Kugelsitz, hingegen drückt die Spiralfeder die Kugel in den Kugelsitz. Die Differenz der beiden Federstärken definiert somit den Schwellwert des Detektors.

Die Vorrichtung zur Begrenzung einer Flüssigkeitsströmung stellt somit ihren Flusswiderstand digital zwischen zwei Zuständen, hoher Flusswiderstand und niedriger Flusswiderstand, ein. Somit weist sie den Nachteil auf, die Stärke eines Flusswiderstands zwischen zwei Zuständen einer Verstellbarkeit zu unterwerfen, ohne die Größe eines Volumenstroms konstant zu halten. Sowohl die Größe des Durchlasses des primären Flussweges als auch die Größe des Durchlasses des sekundären Flusses sind werksseitig durch die Auslegung der Vorrichtung vorgeben.

Die US 2014/0276348A1 (Depuy-Synthes Products, Inc.) aus dem Jahr 2013 lehrt ein Überspannungsschutzgerät, das auf dem Prinzip des sogenannten "*Siphon Guard*^{®}" basiert. Dieser umfasst ein Gehäuse mit einem Einlass und einem Auslass sowie einem ersten Strömungspfad innerhalb des Gehäuses. Der erste Strömungspfad verbindet den Einlass mit dem Auslass. Zusätzlich umfasst das Gehäuse einen zweiten Strömungspfad, der ebenfalls den Einlass und den Auslass verbindet. Beide Strömungspfade weisen jeweils einen Strömungswiderstand bzw. Flusswiderstand auf. Im Vergleich ist der Strömungswiderstand des zweiten Strömungspfades größer als der Strömungswiderstand des ersten Strömungspfads. Innerhalb des ersten Strömungspfads ist ein Ventil mit einem Ventilsitz sowie eine erste Ventilkugel und eine zweite Ventilkugel vorgesehen. Die erste Ventilkugel ist beweglich zwischen einer Verschlussposition, in der die erste Ventilkugel in Kontakt mit dem Ventilsitz ist, und einer Öffnungsposition, in der die erste Ventilkugel vom Ventilsitz beabstandet ist, gelagert. Dabei ist die erste Ventilkugel zwischen einer zweiten Ventilkugel und dem Ventilsitz angeordnet und die zweite Ventilkugel zwischen einer Verschlussposition und einer Öffnungsposition beweglich angeordnet.

Vorteilhafterweise wird der Ventilöffnungsdruck, die Gewichtskraft beider Kugeln im Verhältnis zur Auflagefläche der ersten Kugel im Ventilsitz, durch die Lage beider Kugeln im Gravitationsfeld der Erde angepasst. Je größer der Winkel zwischen einer Vertikalen und der Vertikalachse des Ventils ist, desto geringer ist die Gewichtskraft beider Kugeln im Verhältnis zur Auflagefläche der ersten Kugel im Ventilsitz. Somit sinkt der Ventilöffnungsdruck mit einem Übergehen des Patienten mit dem Ventil aus einer Vertikal- in eine Horizontallage.

Die ausschließliche Bindung des Ventilöffnungsdruckes an die Ventilausrichtung im Gravitationsfeld der Erde Ist von Nachteil.

Auch das Überspannungsschutzgerät hat einen Nachteil: der Flusswiderstand des zweiten Strömungspfades ist werkseitig durch dessen Aufbau vorgegeben. Die Parameter des Flusswiderstands, wie zum Beispiel die Anzahl an Gewindegängen und deren Gewindehöhe sind nach Implantation nicht einstellbar.

Auch die EP 13310192 lehrt ein flusskontrolliertes Gerät (Codman). Diese in der Veröffentlichungsschrift selber als *Anti Siphon* Shunt bezeichnete Vorrichtung lehrt gemäß der Differenzierung nach Miethke ein sich selbst einstellendes flusskontrolliertes Ventil aber kein einstellbares Ventil. Der Anti Siphon Shunt zum Regulieren eines Volumenstroms in einem Patienten umfasst ein Gehäuse, das eine Fluidkammer definiert, sowie eine Einlassöffnung und eine Auslassöffnung. Die Einlassöffnung dient einer Passage eines Fluides in die Fluidkammer, die Auslassöffnung dessen Freisetzen. Zusätzlich umfasst der Anti Siphon Shunt einen Ventilmechanismus zum Regulieren des Fluidflusses durch die Fluidkammer auf Grund des über ihr anliegenden Druckgradienten. Dazu weist der Ventilmechanismus in der Fluidkammer eine Barriere auf, die eine Öffnung vorweist durch die Fluid passieren kann. Ferner umfasst der Anti Siphon Shunt einen Drucksensor zur Detektion des externen Druckes, der die Fluidkammer umgibt sowie ein Vorspannelement, z.B. eine Feder. Dieses steht in Wirkverbindung mit dem Drucksensor und bezweckt ein Aufbringen einer ersten Kraft gegen eine erste Oberfläche einer Kugel. Dadurch wird die Kugel gegen die Öffnung gedrückt, so dass eine Passage des Fluids durch die Barriere folgerichtig durch die Fluidkammer verhindert wird. Eine Ausgleichskraft wirkt auf eine zweite Oberfläche der Kugel in entgegengesetzter Richtung der ersten Kraft. Sowohl die erste als auch die zweite Oberfläche sind dabei annähernd gleich groß.

Die Kugel soll die Öffnung in der Barriere mittels einer Kugel verschließen, bis ein Öffnungsdruck erreicht ist, der das Verhältnis aus der Differenz zwischen der ersten Kraft abzüglich der Ausgleichskraft übersteigt.

In weiterer Ausführung lehrt die Schrift eine zweite Technik, um ein Ende des Vorspannelementes, der Feder zu verschieben, so dass sich deren Vorspannkraft ändert. Dazu schlägt die Schrift vor die Peritonealhöhle, auch als Bauchfellhöhle bezeichnet, mit der Fluidkammer durch einen ersten Kanal zu verbinden. Dieser kann zum Beispiel ein Schlauch sein. Der Vorschlag beinhaltet weiter eine Referenzkammer, welche ebenfalls mit der Peritonealhöhle über einen zweiten Kanal verbunden ist. Fluidkammer und Referenzkammer sind über eine Membran miteinander verbunden, die Membran ist mit einem Ende des Vorspannelements, der Feder verbunden. Durch diese Verbindung wird die Vorspannung des Vorspannungselements verändert, sobald die Membran auslenkt. Die Auslenkung folgt dabei dem Druckunterschied zwischen Peritonealhöhle und Referenzkammer. Der Anti-Siphon Shunt stellt seinen Öffnungsdruck daher durch eine Steifigkeitsanpassung eines Vorspannelements selbstständig ein.

Diese Schrift lehrt aber keinen Weg, um einen Durchlass, wie zum Beispiel einen Spalt zwischen einer Barriere und einer Kugel, einzustellen.

Im Stand der Technik sind damit folgende Merkmale flussreduzierender Ventile zur Therapie eines Hydrozephalus bekannt,
A.) ein Gehäuse, mit einem Einlass und einem Auslass, dass
B.) mindestens ein Flussweg umfasst, der durch das Gehäuse verläuft,
C.) wobei das Gehäuse eine Barriere und einen Körper aufweist, der die Barriere öffnen und verschließen kann.

Dem Stand der Technik ist damit der Nachteil gemein, die Ventrikelgrößen und deren Zustand außer Acht zu lassen. Dadurch vernachlässigt der Stand der Technik die Bedeutung eines abgeführten Drainagevolumens an Liquor verschiedener Patienten. Die Nachgiebigkeit, die sogenannte Compliance beschreibt in der Physiologie die Dehnbarkeit von Körperstruktur. Im Anwendungsgebiet des Hydrocephalus entspricht dies der Nachgiebigkeit der Ventrikel. Da die Ventrikel naturbedingt patientenabhängig sowohl in ihrer Geometrie als auch in ihren Zustand unterschiedlich sind, ist es auch deren Nachgiebigkeit. Die Nachgiebigkeit der Ventrikel verhält sich proportional zu ihrer Volumenänderung und umgekehrtproportional zu ihrer Druckänderung. Wenn die Nachgiebigkeit patientenabhängig ist, dann variiert in deren Abhängigkeit die Druckantwort bei gleich abgeführten Drainagevolumen.

US 2014 0336 560 (Hakim Carlos) lehrt einen programmierbaren Shunt mit einem magnetischen Rotor. Der Rotor ist mit einer Kurvenscheibe verbunden. Auf der Kurvenscheibe liegt eine Zunge eines Biegeglieds auf, so dass eine Rotation/Verschwenken des Rotors ein Wandern der Zunge entlang der Kurvenbahn folgt. Weil die Kurvenbahn eine Steigung aufweist, wird die Zunge durch die Rotation/Verschwenken angehoben oder herabgelassen. Weil die jeweilige Höhe der Zunge einen Hebel vorspannt, der eine Kugel in ihren Sitz drückt, folgt aus einem Verändern der Vorspannung ein Einstellen des Ventils.

Als nächstkommendem Stand der Technik wird von der US 5 167 615 ausgegangen. Dieser lehrt ein physiologisches Shunt System zum Kontrollieren eines Fluidflusses von einem menschlichen Körperteil zu einem Anderen.

Dazu umfasst er ein Gehäuse, welches zwei Einlasskanäle aufweist, In jedem Einlasskanal ist eine Schließeinheit, also ein Ventil angeordnet.

Die erste Schließeinheit ist ein Ventilstopfen, die zweite Schließeinheit ist eine Klappe. Der Stopfen öffnet den Einlasskanal, wenn ein bestimmter Einlassdruck vorhanden ist. Die Klappe ermöglicht ein flexibles Einstellen eines Spalts, in Anhängigkeit von dessen Größe eine Fluidvolumen von ihm durchflossen werden kann. Weil der Einlassdruck durch den Ausbildung des Stopfens mitbestimmt ist, ist er im Gebrauch, also nach einer Implantation nicht einstellbar. Wird im Gebrauch der Spalt eingestellt, bestimmt dieses den Einlassdruck des Ventils

Die Klappe ist dazu vor einem offenen Einlasskanalende drehbeweglich gelagert. Weil die Klappe an ihrem ersten Ende rotatorisch losgelagert ist, ist ihre Stellung zum offenen Einlasskanalende durch einen Winkel zwischen der Klappe und dem offenen Einlassende beschrieben. Ist der Betrag des Winkels null, ist die das Einlasskanalende verschlossen. Je größer der Betrag des Winkels ist, desto geöffneter ist das Einlasskanalende. Weil ein Verschließen und Öffnen aus einer Rotation der Klappe resultiert, ist diese mittels eines Glieds an eine Drehscheibe verbunden, so dass aus deren Verdrehen in einem Öffnen und Schließen folgt. Die Verbindung des Glieds an eine Drehscheibe ist durch einen Bolzen umgesetzt. Dieser ist an seinem einen Ende in das Glied eingebracht und läuft mit seinem zweiten Ende in einem Schlitz. Sein Lauf im Schlitz zwingt ein zweites Ende der Klappe, das dem Einlasskanalende zugewandte Ende, auf eine Bewegungsbahn. Weil die Drehscheibe magnetisiert ist, kann sie mittels eines separaten Magneten verdreht werden.

Im Gegensatz zur drehbeweglichen Lagerung der Klappe, ist der Ventilstopfen unidirektional in einem Sitz gelagert. Seine Stellung, entweder geöffnet oder verschlossen wird durch einen Druck im Einlasskanal mitbestimmt. Ab einem bestimmten Druck, dem Ventilstopfenöffnungsdruck, öffnet er. Die Schließeinheit gibt folglich einen Fluss durch sie frei. Der Öffnungsdruck wird auch durch dessen Verbau im Gehäuse mitbestimmt, da dieser im Gebrauch des Ventils, also nach Implantation nicht mehr geändert werden kann, ist die erste Ventileinheit nicht einstellbar.

Auch im Auslasskanal des Ventils ist eine Schließeinheit vorgesehen. Sie entspricht in Aufbau und Funktion der ersten Schließeinheit, ist also auch im Gebrauch nicht mehr einstellbar

Klappe und Ventilstopfen, beide Verschließeinheiten sind mit dem Auslasskanal des Ventils verbunden.

Erster Nachteil des nächstkommenden Stands der Technik ist, dass dessen Lehre ein genaues Einstellen einer Abflussgeschwindigkeit verhindert. Die Lehre setzt zweite Schließeinheiten, zwei Differenzdruckschließeinheiten und eine zwischen ihnen angeordnete erste, drehbewegliche Schließeinheit mit Klappe voraus. Ein Öffnen und Schließen der Klappe variiert zwar einen Volumenfluss durch die Schließeinheit, beeinflusst damit zwar den im Strömungspfad hinter ihr liegende Öffnungsdruck der zweiten Schließeinheit, öffnet diese aber nicht zwangsläufig. Somit ist das Einstellen einer vorgesehenen Abflussgeschwindigkeit ungenau.

Diese Ungenauigkeit wird durch ein von der Lehre vorgeschlagenes Getriebe noch erhöht. Das Getriebe umfasst die Drehscheibe mit Führungsschlitz, einen darin laufenden Bolzen (Cam Rider), ein Kupplungsglied sowie einen ihn kuppelndes Verschlussglied (Plug). Jeweils zwei der fünf Glieder sind miteinander tolerant, so dass sich eine Gesamttoleranz für das Getriebe kumuliert. Weil die Lehre als Getriebe einen Drehverschluss vorschlägt, wird sie noch erhöht. Ein sicherer Lauf eines Bolzens in einem Schlitz setzt voraus, dass der Schlitz einen hinsichtlich seiner Toleranz einen Kompromiss auf Lauffreiheit und Laufführung leistet. Der Kompromiss an Lauffreiheit erhöht die Toleranz.

Nach der Lehre des nächstkommenden Stands der Technik ist aus Plastik zu fertigen. Plastik ist ein elastisches Material, auf Grund dessen es kaum Grundlage hochpräziser Teile von Ventilen sein sollte. Die Elastizität von Plastik trägt zur Ungenauigkeit der Einstellung des Stands der Technik bei.

Ein zweiter Nachteil des Stands der Technik liegt im Risiko seiner Lehre begründet. Dieses resultiert aus einem möglichen Anlegen des Verschlussglieds (Plug) an das offene Ende des Einlasskanals. Liegt das Verschlussglied an, ist der Einlasskanal verschlossen. Weil er verschließbar ist, ist eine Schaltfunktion gegeben. Unbesonnenes, falsches oder missverstandenes Einstellen des Ventils führt zu einem Abschalten des Durchflusses mit den daraus folgenden Konsequenzen für Patienten. Dieses Risiko vergegenwärtigt sich durch die Betrachtung der Einstellungsdimensionen der Lehre. Ein präzises Einstellen erfordert ein Positionieren der Klappe vor dem Einlasskanal im metrischen Minimalbereich. Umgekehrt resultiert das Risiko durch eine minimale Einstellungsänderung den Einlasskanal freizugeben, so dass eine ungewollte Abflussgeschwindigkeit folgt.

Ein dritter Nachteil der Lehre folgt aus einer Kombination von drei Schließeinheiten, sie macht die Lehre unnötig kompliziert. Allen drei Schließeinheiten ist gemein, als Schaltfunktion dienen zu können. Hinsichtlich eines Abschaltens ist die Lehre somit dreifach redundant. Eine dreifache Redundanz ist kompliziert, sie verhindert ein einfaches Verständnis durch einen Anwender und steht damit einer sicheren Anwendung entgegen.

Die Erfindung hat sich die Aufgabe gestellt, die Ventile zu verbessern. Dabei geht die Erfindung von der Erkenntnis aus, dass Patienten auf einen Abfluss von Liquor mit unterschiedlichem Befinden reagieren. Teilweise wird das Wohlbefinden erheblich beeinträchtigt. Dieser Erkenntnisausgang erhebt den Anspruch vorgenannte Nachteile zu überwinden, um eine Kontrolle von Fluidflüssen von einem menschlichen Körperteil zu einem anderen weiter zu verbessern.

Die Verbesserung wird mit den Merkmalen des Hauptanspruches erreicht wie beschrieben in Anspruch 1. Die Unteransprüche beschreiben bevorzugte Ausführungsbeispiele.

Eine vorteilhafte Ausführungsform eines erfindungsgemäßen Hydrocephalusventils zum Abfluss von Liquor aus Ventrikelsystemen von Patienten sieht mindestens ein Gehäuse mit Gehäuseinnenraum vor, das mindestens einen ersten Durchlass zum Einlassen und/oder Auslassen umfasst, wobei mindestens ein im Gehäuseinnenraum angeordneter Körper mindestens in einer Richtung bewegbar ausgebildet ist und wobei mindestens eine Einstelleinheit vorgesehen ist. Nach der Erfindung kann mit der Einstelleinheit die Abflussgeschwindigkeit im Durchlass eingestellt werden, so dass ein im Ventrikel bestehender Liquor-Überdruck gegenüber einem für den betreffenden Hydrocephalus-Patienten vorteilhaften Liquor-Druck langsam abgebaut wird, ohne das Befinden des Patienten wesentlich zu stören. Beispielsweise kann der gewünschte Liquordruck im Ventrikel 20mm WS(Wassersäule) (entspricht 1,9613 mbar) sein und der Überdruck auch 20mm WS (entspricht 1,9613 mbar) sein. Nach der Erfindung findet dann eine begrenzte Drainage des überschüssigen Liquors statt.

Nach der Erfindung wird mit einer Drainageleistung (Liquorvolumen pro Zeiteinheit) drainiert. Dazu wird die Drainageleistung auf maximal 1000 ml/h (Milliliter pro Stunde) begrenzt, Die Begrenzung ist abhängig vom Wohlbefinden des Patienten. Dabei können sich auch folgende Obergrenzen ergeben:
- 900 ml/h
- 800 ml/h
- 700 ml/h
- 600 ml/h
- 500 ml/h
- 400 ml/h
- 300 ml/h
- 200 ml/h
- 100 ml/h

Vorzugsweise beträgt die Obergrenze 200 ml/h, noch weiter bevorzugt 100 ml/h und höchst bevorzugt 50ml/h.

Die Untergrenze ist gleichfalls abhängig vom Wohlbefinden. Darüber hinaus kann aus medizinischen Gründen eine schnellere Drainage geboten sein. Solange medizinische Gründe nicht eine schnellere Drainage gebieten, ist die Untergrenze 1 ml/h vorzugsweise 1 ml/h, noch weiter bevorzugt mindestens 3 ml/h und höchst bevorzugt mindestens 5 ml/h. Auch Untergrenzen von mindestens 10 ml/h oder 30ml/h kommen in Betracht. Gleichwohl können sich folgende Bereiche für die Drainageleistung ergeben:
1 bis 30 ml/h oder 5 bis 200 ml/h oder 10ml/h bis 200 ml/h
oder 30mlh bis 400ml/h

Die jeweiligen Grenzen der Drainageleistung bilden sich in der Einstelleinheit ab.

Die Einstelleinheit kann nach der Erfindung die Öffnung so fein einstellen, daß die gewünschte Drainageleistung erreicht wird. Die nachfolgend erläuterten Ausführungsformen geben die baulichen Einzelheiten wieder.

Mit dem erfindungsgemäßen Hydrocephalusventil lässt sich überraschender Weise das Wohlbefinden von Patienten steigern.

Das Ventrikelsystem eines jeden Menschen variiert in seiner Größe im Vergleich zu anderen Menschen. Während ein erster Patient ein kleinvolumiges Ventrikelsystem hat, sog. Schlitzventrikel, hat ein zweiter Patient ein weites Ventrikelsystem.

Auf Grund dieser Größenvarianz folgt aus einer Hydrocephalustherapie beider Patienten mit einem identischen Hydrocephalusventil eine unterschiedliche Konsequenz. Einem Abfluss eines definierten Liqourvolumens, z.B. eines Tropfens folgt eine unterschiedliche Druckveränderung im Ventrikelsystem beider Patienten. Ist ein Abfluss, z.B. eines kleinen Liqourvolumens für einen Patienten mit kleinvolumigem Ventrikelsystem beachtlich - er verspürt Unwohlsein-, ist der gleiche Abfluss für einen Patienten mit großvolumigem Ventrikelsystem unbeachtlich.

Summarisch ermöglicht die Erfindung somit ein patientenbezogenes individuelles Einstellen eines Abflusses oder dessen Geschwindigkeit.

Wahlweise ist eine erfindungsgemäße Drainage allein mit obiger Einstellung versehen. Vorzugsweise wird die Drainageleistung dann so gewählt, daß im Unterschied zu herkömmlichen Drainagen eine ständige Drainage erfolgt. Zugleich aber eine Überdrainage verhindert wird. Dann ergibt sich eine Tropfendrainage. Dazu aber eine Überdrainage verhindert wird. Dann ergibt sich eine Tropfendrainage. Dazu kann zum Beispiel das pro Zeiteinheit anfallende Liquorvolumen ermittelt werden, indem der überschüssige Liquor zunächst über eine längere Zeit (mehrere Tage) nach außen abgezogen wird und mit einem Meßbecher bestimmt werden kann.

Danach läßt sich aus der gesammelten Liquormenge ein mittlerer Liquoranfall/Drainagemenge pro Zeiteinheit ermitteln. Der Wert kann dann bei der erfindungsgemäßen Ventileinstellung übernommen werden.

Alternativ kann eine solche Einstellung im Wege von Näherungsversuchen unter Messung des Ventrikeldruckes finden. Dabei wird der Patient mit Druckmessungen begleitet und werden die Einstellungen am erfindungsgemäßen Ventil solange verändert, bis sich im Ventrikel ein üblicher Druck installiert hat.

Nach der Erfindung können die zur Einstellung der Drainage bewegten Ventilkörper sowohl geradlinig als auch auf einer Kurvenbahn bewegt werden.

Vorzugsweise findet eine geradlinige Bewegung des Ventilkörpers statt.

In einem anderen vorteilhaften Ausführungsbeispiel der Erfindung wird mindestens ein Hydrocephalusventil nach mit mindestes einem zweiten Ventil, welches hinter oder vor das erfindungsgemäße Hydrocephalusventil geschaltet ist, kombiniert. Das eine Ventil kann dabei ein herkömmliches Ventil mit einer "auf" und "zu"-Funktion sein. Das erste Ventil hat dann die Aufgabe, eine Überdrainage zu verhindern. Das zweite Ventil kann sich dann auf die erfindungsgemäße Beschränkung des Durchflusses konzentrieren.

Beide Ventile können dabei in einem Gehäuse angeordnet sein oder separate Gehäuse aufweisen.

Eine Ventilkombination erhöht die Chancen auf einen Behandlungserfolg, denn sie ermöglicht ein Abstimmen einer Kombination des Ventils mit dem Hydrocephalusventil nach Anspruch 1 auf den jeweiligen Anwendungsfall. Die Ventilkombination bringt also den Vorteil hervor, flexibel auf die Behandlungsbedürfnisse eines Patienten reagieren zu können.

Vorzugsweise wird für die erfindungsgemäßen Ventile das Prinzip eines Körpers in einem (Öffnungs-)Spalt. Das Prinzip der Erfindung kann Liquor strömungsgerecht in den Spalt eintreten.

Die bessere Liquorführung gibt der erfindungsgemäßen Anordnung auch Sicherheit gegen Ablagerungen.

Das erfinderische Hydrocephalusventil ist zum Beispiel mit einem Gravitationsventil kombinierbar. Das Gravitations-Ventil besitzt ein Schließteil, üblicherweise eine Kugel, das in der Stehend-Lage des Patienten aufgrund entsprechender Anordnung im Patienten die Drainageleitung unter dem Gewicht des Schließteiles verschließt. In der Liegend-Lage des Patienten öffnet das Ventil schon bei geringem Liquordruck, der das Schließteil in die Offen-Stellung verschiebt. Solche Ventile öffnen ganz oder schließen ganz.

Es sind auch Gravitations-Ventile mit zwei Kugeln bekannt, von denen die eine klein und die andere groß ist. Die kleinere Kugel bewirkt in der Schließstellung die Abdichtung im Ventilsitz. Die große Kugel dient der Erhöhung des Gewichtes in der Schließstellung.

Das erfinderische Hydrocephalusventil ist auch mit mindestens einem Differenzdruckventilventil kombinierbar. In diesem Fall kann das Differenzdruckventil als Schalter herangezogen werden. Die einstellbaren Differenzdruckventile besitzen üblicherweise ein federbelastetes Schließteil, zumeist eine federbelastete Kugel. Bei Überschreiten eines bestimmten Liquordrucks öffnet sich das Schließteil. Die Öffnung vergrößert sich mit zunehmendem Druck gegen den Widerstand der auf dem Schließteil lastenden Feder. In der Liegend-Lage ist der Liquordruck am größten; demzufolge die Öffnung und die Drainage am größten. In der Stehend-Lage ist der Liquordruck am geringsten, demzufolge die Ventilöffnung am geringsten. Differenzdruckventile haben den Vorteil einer stufenlosen Anpassung an Zwischendrücke in Positionen zwischen der Stehend-Lage und der Liegend-Lage. Die Einstellbarkeit solcher Ventile hat darüber hinaus den Vorteil der Anpassung an unterschiedliche Drainagebedürfnisse. Bei unterschiedlichen Patienten sind unterschiedliche Drainagebedürfnisse normal. Aber auch an Einzelpersonen kommt eine Änderung der Einstellung vor. Das ist regelmäßig nach dem Implantieren der Fall, bis die richtige Drainage für das Krankheitsbild gefunden ist.

Die Ventilkombination kann auch eine Mehrzahl von Ventilen umfassen. In der Kombination eines Gravitationsventils mit einem Differenzdruckventil tritt das Differenzdruckventil in der Liegend-Lage in Funktion. In der Liegend-Lage ist das Gravitationsventil offen. In der Situation regelt das Differenzdruckventil die Drainage. Das Gravitationsventil kann in Kombination der beiden Ventile dem anderen Ventil in Strömungsrichtung des Liquors im Gehäuse vorgeordnet oder nachgeordnet sein.

Vorzugsweise wird das erfindungsgemäße Ventil nach einer Einstellung in der betreffenden Stellung gesichert, damit keine unbeabsichtigte Verstellung erfolgen kann. Eine geeignete Sicherung wird durch eine Bremse gebildet, die vor jeder Einstellung gelöst wird und nach einer Änderung der Einstellung selbsttätig die Bremsstellung/Sicherungsstellung einnimmt. Dadurch ergeben sich folgendebei der Einstellung folgende Schritte: Verbringen mindestens eines Magneten über das Hydrocephalusventil; Lösen einer Bremse einer Einstelleinheit des Hydrocephalusventils; Verdrehen der Einstelleinheit, so dass der Körper in einer vorgesehenen Position im Spalt im Hydrocephalusventil angeordnet wird.

Weil der Körper in eine vorgesehene Position gebracht werden kann, bietet das Verfahren den Vorteil die Abflussgeschwindigkeit einzustellen. Durch diese Einstellbarkeit kann die Druckänderung im Ventrikelsystem jedes Patienten unabhängig von der Beschaffenheit des Ventrikelsystem eingestellt werden. Wenn zwei Patienten unterschiedliche Ventrikelgrößen aufweisen, kann durch ein unterschiedliches Einstellen, also Positionieren des Körpers im Durchlass eine unterschiedliche Abflussgeschwindigkeit eingestellt werden, so dass die Druckantworten in den Ventrikelsystemen der Patienten gleich sind. Unabhängig von den Ventrikelgrößen ermöglicht die Erfindung somit ein Einstellen einer, nämlich der gleichen Druckantwort für unterschiedliche Patienten.

Vorzugsweise ist der Körper in mindestens einem ersten Durchlass angeordnet. Dabei können unterschiedliche Körper Anwendung finden, zum Beispiel:
- Körper mit größerem Durchmesser als der Durchlass
- Körper mit kleinerem Durchmesser als der Durchlass
- Körper mit Rundung an dem Ende, das dem Durchlass zugewandt ist
- Körper mit konischer Spitze an dem Ende, das dem Durchlasse zugewandt ist
- Körper mit keilförmiger Spitze an dem Ende, das dem Durchlass zugewandt ist
- stopfenförmiger Körper
- stabförmige Körper mit runden und/oder eckigen Querschnitten
- profilförmige Körper mit Einformungen und/oder Ausformungen im Querschnitt

Vorzugsweise findet ein Körper mit konischer Spitze und kleinerem Durchmesser als der Durchlass Anwendung.

Die Körper können mit unterschiedlichen Durchlassöffnungen korrespondieren, zum Beispiel:
- an der Berührungsfläche mit dem Körper scharfkantigen und/oder gerundete und/oder glatten Öffnungen
- sich konisch erweiterenden Öffnungen
- sich keilförmig erweiternden Öffnungen
- Öffnungen ohne Berührungsfläche mit dem Körper
- Öffnungen mit Führungen für den Körper

Wenn der Körper in seinen Maßen kleiner ist als der Durchlass, bildet sich zwischen beiden mindestens ein Spalt aus. Auch diese Spaltbildung kann zur erfindungsgemäßen Einstellung genutzt werden.

In weiterhin vorteilhafter Ausgestaltung wird als Körper mindestens ein geführter Stopfen, Keil, Konus, Profilstab oder eine Kugel verwendet.

Unterschiedlichem Liquoranfallkann wahlweise unterschiedlicheGeometrien am Körper und der zugehörigen Duchlass-Öffnung zur Folge haben. So sind zum Beispiel Geometrien der Körper und Durchlass-Öffnung besonders für geringen Liquoranfallgeeignet, bei denen die Körperbewegung

Wenn ein zylindrischer Profilstab als Körper, also als Schließteil vorgesehen ist, und wenn dieser mit einer konischen Spitze in eine Gehäusebohrung mündet, resultiert vorteilhafterweise ein präzise einstellbarer Verschlussmechanismus. Die konische Spitze definiert darin im Zusammenwirken mit der Gehäusebohrung einen für die Drainageströmung maßgeblichen Öffnungsquerschnitt der Gehäusebohrung.

Von Vorteil ist, wenn die Längsachse des zylindrischen Profilstabs mit der Mittelachse der den Einlass oder Auslass bildenden Gehäusebohrung fluchtet. Wahlweise sind in dem Gehäuse Einsätze für den Einlass und/oder Auslass vorgesehen. Die Bohrungen für den Einlass und/oder Auslass sind dann in dem Einsatz vorgesehen.

Vorzugsweise weist der Körper einen Kragen auf, wobei zwischen dem Kragen und mindestens einer Teilfläche der Einstelleinheit eine Feder vorgesehen ist, welche die bleibende Berührung des Körpers mit der Teilfläche sicherstellt. Durch diese Sicherstellung ist eine Einstellfähigkeit der Erfindung über die Zeit gegeben.

Vorteilhafterweise stützt der Kragen die Feder, so dass deren Federkraft den Körper, z.B. eine Nadel aus einer Bohrung herauszudrücken sucht. Kragen und Feder unterstützen somit ein Prinzip, dessen Ziel ist, die Erfindung jederzeit in einem geöffneten Zustand zu halten. Durch dieses Prinzip wird einer Gefahr eines ungewollten Verschließens vorgebeugt. Verschließt ein Ventil ungewollt, fließt kein Liquor mehr ab, die Symptome eines Hydrocephalus bleiben unbehandelt.

Weiter ist von Vorteil, wenn im Durchlass mehr als ein einstellbarer Körper angeordnet ist, zum Beispiel am Einlass ein Körper und am Auslass ein Körper.. In Betracht können auch Ventilkörper in sogenannter Parallelschaltung kommen.Wenn die beiden Körper dadurch klein ausgeführt werden können, sogar kleiner als ein ihnen in der Wirkung identischer einzelner Körper, kann das Bauvolumen der Ventil-, ein-, und Auslässe kleiner gefertigt werden, so dass das Ventilbauvolumen verkleinert wird.

Von Vorteil ist, wenn der Durchlass mindestens ein erster Ventilauslass ist, der einen Liqourstau innerhalb des Gehäuses vermeidet. Ist der Durchlass ein erster Ventilauslass, sitzt der Körper also in einem Ventilauslass und bilden Körper und Ventilauslass einen dauerhaften Spalt, ist die Erfindung auf Seiten des Auslasses dauerhaft geöffnet. Liqour wird folgerichtig dauerhaft abgeleitet, dessen Stau verhindert.

Weiter ist von Vorteil, wenn die Einstelleinheit eine Kurvenscheibe umfasst oder ist. Dann wird eine Stellvorrichtung, die Einstelleinheit gebildet deren Stellgröße der Kurvenverlauf ist. Wird die Kurvenscheibe aus einem zeitüberdauernden Material, wie Titan ausgeformt, folgt eine langlebige Stellvorrichtung. Deren Langlebigkeit erfüllt vorteilhafterweise die Forderung einmaliger Implantation. Eine Mehrfachimplantation wird mit hoher Wahrscheinlichkeit vermieden.

In einer besonders vorteilhaften Ausführungsform ist die Kurvenscheibe ein Rotor.

Der Rotor/Kurvenscheibe kann gestuft ausgeführt sein. Dadurch weist er eine Mehrzahl an Kurvenbahnen auf. Jede Kurvenbahn kann zur Steuerung unterschiedlicher Ventile verwendet werden. Ein erstes Schließteil zum Schließen des Ventils gegen die Einlassöffnung oder Auslassöffnung kann über eine erste Kurvenbahn gesteuert werden. Ein zweites Schließteil kann über eine zweite Kurvenbahn gesteuert werden.

Vorteilhaften weist die Kurvenscheibe, ein Rotor Auswölbungen und Einbuchtungen auf. Ein An- und Abschalten wird durch entsprechende Ausbuchtungen und Einwölbungen des Rotors/Kurvenscheibe erreicht. Die Ausbuchtung drückt dabei eine Kugel aus einem Sitz. Die Einwölbung lässt den Sitz einer Kugel zu. Somit wird vorteilhafterweise die Funktion eines Schalters erreicht.

Wahlweise ist auch ein stufenweises Schalten, also Freigabe vorgesehen. Die Schaltstufen können eine schrittweise Öffnung des Ventils beinhalten. Damit lässt sich auch bei größeren Drainageleitungen mit an sich stärkerer Liquorströmung eine kleinere Liquorströmung einhalten.

Bei Verwendung eines Rotors/Kurvenscheibe als Verstelleinrichtung für das Gravitationsventil kann es bei Kombination mit einem zweiten Ventil im gemeinsamen Gehäuse zu einer Kombination mit einer weiteren Kurvenscheibe oder einem Rotors kommen, wenn auch das zweite Ventil mit einem Rotor/Kurvenscheibe verstellbar ist. Von Vorteil ist, die beiden Rotoren/Kurvenscheiben dann gemeinsam zu verstellen. Zur Verstellung eignen sich unter anderem die bekannten Magnete in dem Rotor/Kurvenscheibe in Verbindung mit bekannten Verstelleinrichtungen, die über dem Gehäuse auf der Haut des Patienten aufgesetzt werden und ihrerseits mit Magneten versehen werden, so dass über ein Verdrehen der Verstelleinrichtung die Rotoren/Kurvenscheiben verschwenkt werden können.

Von Vorteil kann sein, jeden der Rotoren/Kurvenscheiben separat herzustellen und den Bedürfnissen des Patienten anzupassen und anschließend in der richtigen Stellung zueinander miteinander zu verbinden, um beide Rotoren/Kurvenscheiben gemeinsam zu verstellen. Dabei bestimmt der Bereich der Einbuchtung in der zum Gravitationsventil gehörigen Kurvenscheibe/Rotor die Zuschaltung des Gravitationsventils bzw. die Drainage des Liquors. Innerhalb dieses Bereiches soll das zweite Ventil seine Wirkung entfalten. Deshalb soll die zum zweiten Ventil gehörige Kurvenscheibe die gewünschte Stellung in Bezug auf das zweite Ventil einnehmen.

Eine bevorzugte Ausführungsform zeichnet sich dadurch aus, dass die Kurvenscheibe eine Achse aufweist, wobei die Achse vor dem Durchlass angeordnet ist.

Durch das Positionieren des Drehpunkts des Rotors, bzw. einer Kurvenscheibe vor dem Durchlass wird ein unwuchter Lauf des Rotors vermieden. Dieses wird noch verbessert, wenn die Drehachse auf der Symmetrieachse eines Durchlasses liegt. Weil keine Unwucht, ein Abstand zwischen Symmetrieachse und Drehachse vorliegt, wird der Körper über die Kurvenbahn der Kurvenscheibe bzw. des Rotors präzise, d.h. parallel im Durchlass geführt.

Vorzugsweise umfasst die Einstelleinheit mindestens einen Rotor. Der Rotor ist ein drehbarer/schwenkbarer Teil eines Hydrocephalusventil aufgefasst werdenDie Rotoren können unterschiedliche Form aufweisen: rund, oder haben die Form einer Scheibe, einer Teilscheibe, einer Schecke, einer Teilschnecke oder eines Gewindes.

Weiter ist von Vorteil, wenn die Einstelleinheit bzw. der Rotor mindestens einen Magneten umfasst. Magnetkräfte durchdringen die Haut von Patienten. Vorteilhafterweise kann dann mittels eines Werkzeugs, welches ebenfalls mindestens einen Magneten aufweist, der Rotor der Einstelleinheit gedreht bzw. verschwenkt werden.

Vorteilhafterweise können auf diesem Wege auch Schwenkarme, Hebel, Federn und auch der Körper der Einstelleinheit mit dem Rotor bewegt werden. Vorzugsweise umfaßt die Bewegung der Einstelleinheit eine Teilrotation und/oder eine Rotation und/oder eine Anzahl an Rotationen und/oder Schwenkbewegungen oder ein Schub und/oder ein Hub. Damit läßt sich der Körper der Einstelleinheit präzise einstellen. Deren Präzision steigt vorteilhafterweise, wenn eine Teil- oder Rotation der Einstelleinheit in eine Linearbewegung des Körpers übersetzt wird. Durch diese Übersetzung können Teilrotationen- oder Rotationen bzw. Schwenkbewegungen der Einstelleinheit, welche für einen Menschen als angenehm einzustellen gelten, in präzise Linearbewegungen übersetzt werden.

Weiter von Vorteil ist, wenn die Einstelleinheit die Bewegung des Körpers entlang einer Kurvenbahn steuert. Kurvenbahnsteuerungen überdauern, sind präzise und einfach herzustellen.

In weiterhin vorteilhafter Ausgestaltung wird die Kurvenbahn durch die Umfangsfläche oder die Stirnfläche der Einstelleinheit oder des Rotors gebildet. Dadurch können Teil- oder Rotationen und Schwenkbewegungen der Einstelleinheit leicht und unkompliziert auf andere Getriebeglieder übersetzt werden.

Vorzugsweise liegt der Körper, also das Schließteil des auslaufseitigen Ventils unter Federdruck an der Kurvenbahn an. Dadurch wird eine instantane Einstellung möglich, jede Änderung der Kurvenbahn resultiert in einer Änderung der Position des Körpers im Spalt.

Weiter ist von Vorteil, dass der Körper an seinem ersten Ende an der Kurvenbahn anliegt. Dadurch wird ein Spiel zwischen Körper und Kurvenbahn verhindert. Jede Änderung einer Einstellung wird sofort auf den Körper übertragen.

In weiterhin vorteilhafter Ausgestaltung liegt der Körper an seinem ersten Ende mit einer Rundung an der Kurvenbahn an. Rundungen können problemlos auf einer Kurvenbahn entlang gleiten, so dass der Verlauf der Kurvenbahn vorteilhafterweise mit geringer Reibung auf die Rundung übertragen werden kann.

Vorzugsweise ragt ein als Profilstab ausgebildeter Körper mit einer Spitze in eine Öffnung des Auslasses, wobei die Spitze vorzugsweise konisch ist, noch weiter bevorzugt der Außendurchmesser des Profilstabes größer als die Öffnungsweite des Auslasses.

Weiter ist von Vorteil, wenn der Auslass eine Rohrform mit zylindrischem Innenmantel besitzt. Dieses ist einfach und präzise zu fertigen.

In weiterhin vorteilhafter Ausgestaltung wird der Auslass durch einen Einsatz des Gehäuses gebildet und die Führung für den Körper durch den Einsatz des Gehäuses gebildet. Einsätze können für unterschiedliche Ausgestaltungen von Ein- und Auslässen bereitgestellt, vorgehalten und während einer Implantation bereitgehalten werden. Dadurch kann ggf. während der Implantation entschieden werden, welcher Körper und welche Ein- oder Auslassdimensionen zu verwenden sind. Durch unterschiedliche Einsätze in Zusammenwirkung mit unterschiedlichen Ein- oder Auslassen kann individuell jeder Patient behandelt werden.

Vorzugsweise ist der Körper an seinem ersten Ende in dem Einsatz abstützt. Das gibt ihm Halt.

In weiterhin vorteilhafter Ausgestaltung der Kombination des erfindungsgemäßen Hydrocephalusventils mit einem weiteren Ventil sind beide Ventile in einem Strömungspfad angeordnet.

Weil sie in einem Strömungspfad liegen, liegt über ihnen ein gemeinsamer Druck an. Dieses hat den Vorteil leichterer Abstimmung der Ventile. Im Gegensatz zum nächstkommenden Stand der Technik ist das erfindungsgemäße Ventil unkompliziert und für Anwender gut verständlich. Ein Strömungskanal erfordert nur dessen Fertigung, dass reduziert Aufwand und Produktionsfehler.

Weiterhin wird eine Vielzahl an strömungsberuhigten Zonen vermieden. Das hat den Vorteil, dass sich dort keine Ablagerungen bilden können.In vorteilhafter Ausführung ist das zweite Ventil der Ventilkombination ein Differenzdruckventil. Dadurch ist die Funktionalität der Ventilkombination durch einen unteren oder oberen Schwellwert begrenzbar.

In weiterhin vorteilhafter Ausgestaltung ist das zweite Ventil ein federbelastetes Schließteil, welches sich in Abhängigkeit von dem Liquordruck schließt und öffnet.

Bei Verwendung eines Gravitationsventils als zweites Ventil wird dieses vorzugsweise durch mindestens eine Kugel, wahlweise durch zwei Kugeln, gebildet. Auch das zweite Ventil kann einen Rotor/Kurvenscheibe besitzen, die unmittelbar mit der das Schließteil bildenden Kugel oder mit einer ein zusätzliches Gewicht bildenden Kugel in Berührung stehen und die als Schließteil bildende Kugel in deren Ventilsitz drücken. Bei Verwendung üblicher Werkstoffe für Kugel, Ventilsitz und Rotor/Kurvenscheibe ist weder an der Kugel, noch an dem Rotor/Kurvenscheibe, noch im Ventilsitz mit nennenswertem Verschleiß zu rechnen. Wenn gleichwohl noch Verschleiß stört, können verschleißfeste Werkstoffe wie Titan für die Kugel, Ventilsitz und Rotor/Kurvenscheibe gewählt werden. Selbst eine geringe Leckströmung zwischen Kugel und Ventilsitz aufgrund eines notwendigen Spieles ist in der Regel unschädlich. Wenn gleichwohl eine geringe Leckströmung stört, kann zum Beispiel der Ventilsitz nachgiebig gestaltet werden. Zusätzlich oder anstelle eines nachgiebigen Ventilsitzes kann auch die Gleitfläche nachgiebig gestaltet werden. Schon eine geringe Nachgiebigkeit reduziert mögliche Leckströmungen. Es können mit der Nachgiebigkeit bei entsprechendem Druck der Ventilkugel im Ventilsitz Leckströmungen auch ausgeschlossen werden.

Von Vorteil ist, wenn ein gemeinsames Gehäuse für beide Ventile vorhanden ist, dann ist nur dieses eine Gehäuse während einer Implantation einzusetzen. Das erspart Aufwand. Wenn das Gehäuse modular aufgebaut ist, kann in Abhängigkeit der Ventrikelgröße des Patienten das Gehäuse weit vor der Implantation vorkonfiguriert werden. Beispielsweise können im Gegensatz zum Stand der Technik individuelle Einsätze für individuelle Ein- oder Auslässe und/oder Körper, Rotoren eingesetzt werden. Auch ein Vorortzusammensetzen an abgelegen Orten wird möglich, so dass unterschiedliche Patientengruppen, wie Kinder oder Ältere durch angepasste Ventile therapiert werden können.

Besonders vorteilhaft ist die Zusammenfassung mehrerer Ventile in einem Gehäuse, vorzugsweise eines Gravitations-Ventils mit einem Differenzdruck-Ventil oder einem anderen Ventil in einem gemeinsamen Gehäuse. Das erleichtert die Implantation. Damit entfällt ein Leitungsstück zwischen den beiden Ventilen des älteren Vorschlages. Es reduziert sich der bauliche Aufwand mit der Zusammenfassung beider Ventile in einem einzigen Gehäuse. Außerdem bietet das Gehäuse die Möglichkeit zur günstigen Gestaltung der Verbindung beider Ventile. Die Verbindung wird durch Kanäle gebildet. Der Kanalquerschnitt kann in dem Ventilgehäuse größer gewählt werden als bei einer verbindenden Leitung des älteren Vorschlages. Außerdem können der Übergang von dem einen Ventil zum verbindenden Kanal und vom verbindenden Kanal zum anderen Ventil (zum Beispiel vom Gravitations-Ventil zu dem verbindenden Kanal und vom verbindenden Kanal zum Differenzdruck-Ventil) strömungsgünstiger gestaltet werden. Dies äußert sich in einem geringeren Strömungswiderstand als bei einer Verbindung der beiden Ventile mit einer Leitung zur Zuführung von Liquor zu dem gemeinsamen Gehäuse und/oder zum Abführen von Liquor..

Weiterhin vorteilhaft ist, wenn in dem Gehäuse das Hydrocephalusventil dem Einlass und das zweite Ventil dem Auslass oder das zweite Ventil dem Einlass und das Hydrocephalusventil dem Auslass zugeordnet ist und dass in dem Gehäuse Kanäle von Hydrocephalusventil zum zweiten Ventil vorgesehen sind, die einen geringeren Strömungswiderstand haben als eine Verbindung beider Ventile mit einer Drainageleitung wie sich für die Zuführung von Fluid zu dem gemeinsamen Gehäuse und/oder zur Ableitung von Fluid von dem gemeinsamen Gehäuse vorgesehen sind.

Bevorzugte Ausführungsformen der Erfindung werden beispielhaft anhand einer Zeichnung erläutert. Die Figuren der Zeichnung zeigen im einzelnen:
Figur 1 eine bevorzugte, erste Ausführungsform der Erfindung in einer schematischen Ansicht von oben,
Figur 1a eine Detailansicht,
Figur 1b eine Detailansicht,
Figur 2 (bestehend aus Fig.2a und 2b) zeigt Einzelheiten einer bevorzugten Ausführungsförm,
Figur 2a zeigt eine Einzelheit mit einer bevorzugten Ausführungsform eines Körpers mit Spalt,
Figur 2b zeigt eine Einzelheit mit einer alternativen Ausführungsform eines Körpers mit Spalt,
Figur 3 (bestehend aus Fig. 3a, 3b, 3c, 3d) zeigt Einzelheiten,
Figur 3a zeigt eine Einzelheit mit alternative Ausführungsform eines Körpers mit Spalt,
Figur 3b zeigt eine Einzelheit mit alternativer Ausführungsform eines Körpers mit Spalt,
Figur 3c zeigt eine Einzelheit mit alternativer Ausführungsform eines Körpers mit Spalt,
Figur 3d eine Einzelheit mit alternativer Ausführungsform eines Körpers in einem Spalt,
Figur 4 zeigt eine alternative, zweite Ausführungsform der Erfindung in einer Seitenansicht,
Figur 5 zeigt eine alternative, dritte Ausführungsform der Erfindung in einer Seitenansicht,
Figur 6 eine alternative, vierte Ausführungsform der Erfindung in einer Ansicht von oben,
Figur 7 eine alternative, fünfte Ausführungsform der Erfindung in einer Seitenansicht,
Figur 8 eine alternative, fünfte Ausführungsform der Erfindung mit einem Rotor und einem magnetischen Kupplungsglied,
Figur 9 verschiedene Druckkurven,
Figur 10 eine bevorzugte, zweite Ausführungsform Erfindung,
Figur 11 eine weitere Ausführungsform der Erfindung in schematischen Darstellung,
Figur 12 eine weitere Ausführungsform der Erfindung in schematischer Darstellung,
Figur 13 eine weitere Ausführungsform der Erfindung in schematischer Darstellung,
Figur 14 eine bevorzugte weitere Ausführungsform der Erfindung in einer schematischen Ansicht von der Seite in geschlossenem Zustand,
Figur 15 eine weitere Ausführungsform der Erfindung in einer schematischen Ansicht von der Seite in geöffnetem Zustand,
Figur 16 eine bevorzugte Ausführungsform in einer Schnittansicht,
Figur 17 eine bevorzugte Ausführungsform in einer Schnittansicht
Figur 18 eine bevorzugte Ausführungsform eines Rotors in einer Draufsicht.

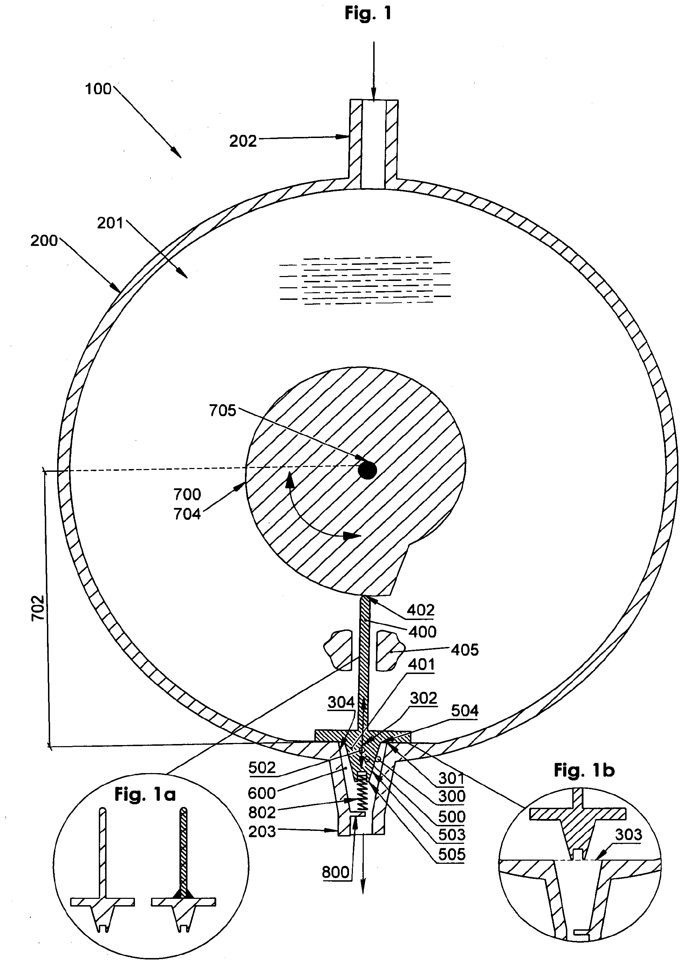

**Figur 1** zeigt ein erfindungsgemäßes Hydrocephalusventil 100 in dessen Aufbau in einer schematischen Ansicht von oben. Sie zeigt einen Aufbau mit einem Gehäuse 200, in dem eine Einstelleinheit 700 in Ausgestaltung einer Kurvenscheibe 704, ein Kupplungselement 400 sowie ein Körper 500 gelagert sind. Ferner umfasst das Gehäuse einen Einlass 202 und einen Auslass 203.

Die Bewegung der Kurvenscheibe 704 ist im Gehäuseinnenraum 201 zentral durch eine Achse 705 zwangsgeführt, hingegen ist der Körper 500 nicht über seine ausgebohrte Körperachse 502, sondern mittels seiner Körpermantelfläche 503 geführt. Figur 1 zeigt, dass der Körper 500 als Keil ausgeformt ist, so das sich dessen Körpermantelfläche 503 von einen ersten Körperendes 504 in Richtung eines zweiten Körperendes 505 verjüngt. Figur 1 zeigt ebenfalls, dass die Form des Durchlass 300 im Querschnitt keilartig bzw. kelchartig ausgeformt ist. Dadurch, dass die Verjüngung der Körper Mantelfläche 503 mit der Verjüngung des Durchlasses übereinstimmt, wird der Keil 506 im Durchlass 300 entlang seiner Körperfläche geführt.

Das Kupplungselement 400 ist mit seinem ersten Ende 401 an den Keil 506 und mit seinem zweiten Ende 402 an die Kurvenscheibe 704 angeordnet. Zweckmäßigerweise kann das Kupplungselement 400 steif oder elastisch sein. Figur 1 zeigt eine Ausführungsform mit steifem Kupplungselement 400. Dieses kann ein Stift oder eine Platte sein. Der Querschnitt durch den Stift kann dabei rund, ellipsoid oder mehreckig sein. In der vorliegenden Ausführungsform ist der Stift ein Rundmetall oder ein Kantmetall aus Titan. Alternativ kann er aber auch aus Edelstahl, einem Duromer oder einem Thermoplast gefertigt sein. Dadurch, dass das Kupplungselement 400 mit der Kurvenscheibe 704 und dem Keil 506 verbunden ist, führt eine Bewegung der Kurvenscheibe 704 die Bewegung des Keils 506.

Durch die Bewegung des Keils 506 im Durchlass 300 resultiert ein Spalt 600 zwischen der Mantelfläche des Keils 506 und der Durchlassinnenfläche 304 des Durchlasses 300. Je weiter der Keil 506 in den Durchlass 300 in Durchlassrichtung 302 hineingeführt wird, desto länger wird der Spalt 600. Mit zunehmender Spaltlänge 602 steigt dessen Spaltinnenfläche. Die Spaltinnenfläche summiert sich aus der Mantelfläche des Keils 506 und der Durchlassinnenfläche 304 vom Beginn des Spalts bis zu dessen Ende. Je größer die Spaltinnenfläche ist und je enger der Spalt ist, desto größer ist die Reibung eines Fluids 900, welches den Spalt 600 passieren soll bzw. passiert mit der Spaltsinnenfläche. Somit wirkt die Mantelfläche des Keils 506 als Flusswiderstand/Strömungswiderstand, also auch der Keil 506. Weil die Position des Keils 506 durch dessen Bewegung im Spalt 600 über das Kupplungselement 400 mittels der Kurvenscheibe 704 einstellbar ist, ist auch der Flusswiderstand/Strömungswiderstand einstellbar. Folglich ist der Flusswiderstand/Strömungswiderstand des erfindungsgemäßen Hydrocephalusventils 100 durch die Einstellbarkeit der Spaltlänge einstellbar.

Über den Flusswiderstand/Strömungswiderstand wird die Durchflussmenge des Fluid pro Zeiteinheit eingestellt.

Eine bewegliche Anordnung des Körper 500 in der ersten Durchlassrichtung 302ermöglicht unterschiedliche, vorteilhafte Varianten zum Herstellen eines Spalts 600. Das kann bedeuten, dass ein Winkel zwischen einer Bewegungsrichtung des Körpers 500, und seiner Bewegung entlang einer Zwangsführung besteht, die zum Beispiel durch ein Linearlager 405 bestimmt wird. Der Winkel zwischen der Hauptbewegungsrichtung des Körpers und der ersten Durchlassrichtung 302 ist in anderen Ausführungsbeispielen kleiner als 80°, vorzugsweise kleiner als 50°, insbesondere kleiner als 20° oder noch kleiner, also kleiner 5° beträgt. Im Ausführungsbeispiel beträgt der Winkel 0 Grad., Also liegt die Hauptbewegungsrichtung des Körpers 500 koaxial zur Durchlassrichtung 302 und bildet sich ein symmetrischer Spalt 600 aus, sobald der Körper 500 vom Durchlass 300 beabstandet wird. Im Ausführungsbeispiel sind die einander zugewandten Abschnitte des Körpers 500 und des Durchlasses 300 symmetrisch. Alternativ zur Spaltlänge 602 und/oder zusätzlich sind andere geometrische Merkmale eines Körpers 500 änderbar, um einen Flusswiderstand/Strömungswiderstand durch den Durchlass 300 zu variieren. In weiterer Ausgestaltung kann die Rauigkeit der Mantelfläche des Keils 506 verändert werden oder deren Verlaufsform.

In einer bevorzugten Ausführungsform ist ein Federsitz 800 in eine Kante des Durchlasses 300 eingearbeitet. Der Federsitz 800 kann dabei ein Stift sein, auf den eine Feder 802, zum Beispiel eine Spiralfeder aufgeschoben werden kann. Der sichere Sitz einer Feder 802 begünstigt deren Positionierung zwischen einer Kante des Durchlasses 300 und dem Keil 506, sodass die Federkraft der Feder 802 den Keil 506 in Richtung des Durchlassendes 301 aus dem Durchlass 300 herausdrückt.

Aufgrund der Rotation oder Schwenkbewegung der Kurvenscheibe 704 und der Folgebewegung des Körpers 500 variiert der Abstand 702 zwischen einem Drehpunkt der Kurvenscheibe 704 und einem Referenzpunkt des Körpers 500.

Neben Kurvenscheiben 704 sind alternativ andere Einstelleinheiten nutzbar, welche ihrer Richtung 701 nach auch translatorisch bewegbar sind.

**Figur 1a** zeigt eine Detailansicht aus Figur 1, welche zwei Alternativen eine Kupplungselements 400 darstellt.

**Figur 1b** zeigt eine Detailansicht aus Figur 1, welche einen Öffnungszustand des Hydrocephalusventils 100 darstellt ( Feder 802 nicht dargestellt). Im geöffneten Zustand berührt das zweite Körperende 505 die Querschnittsfläche 303.

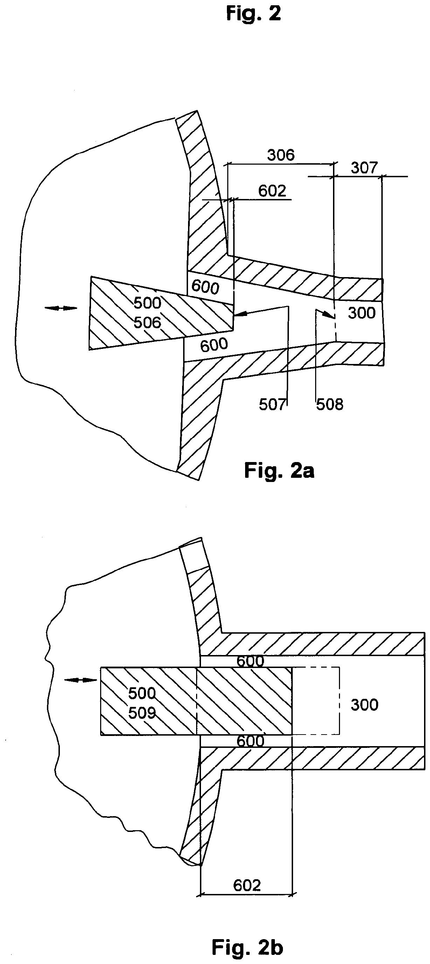

**Figur 2a** zeigt eine Einzelheit mit einer bevorzugten Ausführungsform eines Körpers mit Spalt. Die bevorzugte Ausführungsform basiert auf dem Zusammenspiel zweier Bauteile, einem Durchlass 300 und einem Körper 500.Figur 2a zeigt, dass der Durchlass 300 aus einem trichterförmigen Einlassbereich 306 einen schlauchartigen Abschnitt 307 besteht. Ferner zeigt Figur 2a, dass zwischen den beiden Bauteilen ein Spalt 600 besteht, wenn der Körper 500 als Keil 506 hergestellt wurde. Dazu kann der Keil 506 zum Beispiel aus einem Titan-, Stahl-, oder biokompatiblen Elastomerblock herausgearbeitet sein. In der bevorzugten Ausführungsform ist der Keil 506 aus einem Titanblock herausgefräst. Alternativ kann er aber auch aus einem biokompatiblen Kunststoffblock herausgeschnitten werden. Ein Hinein- oder Herausbewegen des Keils 506 aus dem trichterförmigen Einlass 306 des Durchlasses 300 variiert die Spaltlänge 602 und damit den Flusswiderstand zwischen Keil 506 und Durchlass 300. In einer bevorzugten Ausführungsform dichtet der Keil 506 gegen den Durchlass 300, wenn die umlaufende Kante der Keilstirnfläche 507 gegen die Übergangskante 508 zwischen trichterförmigen Einlass 306 und schlauchartig im Abschnitt 307 anschlägt.

**Figur 2b** zeigt eine Einzelheit mit einer alternativen Ausführungsform eines Körpers mit Spalt. Der Körper 500 hat die Form eines Stabs 509. Je größer die Spaltlänge 602 und je geringer die Spaltweite ist, desto größer ist der

Flusswiderstand/Strömungswiderstand zwischen Stab 509 und Durchlass 300. Das Hineinschieben eines Stabes 509 mit großer Länge resultiert also in einem Erhöhen des Flusswiderstands. Wird der Stab 509 tief genug in den Durchlass 300 hineingeschoben folgt damit ein unendlich hoher Flusswiderstand. In der vorliegenden Ausführungsform ist die Länge des Stabes 509, also die maximale Höhe des Flusswiderstands an die lageabhängige Gewichtskraft eines Volumens an CSF im Gravitationsfeld der Erde angepasst. Alternativ kann der Körper 500 eine Kugel (nicht dargestellt), ein Konus (nicht dargestellt) oder ein Zylinder (nicht dargestellt) sein.

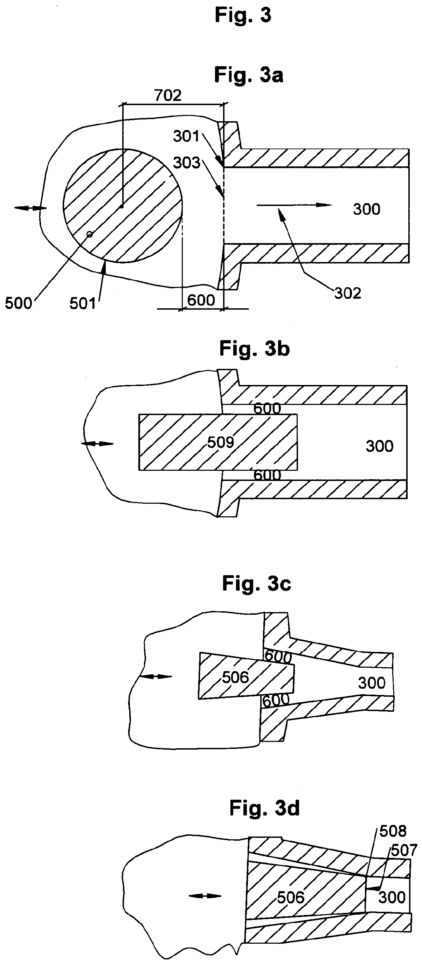

**Figur 3a** zeigt eine Einzelheit mit alternativer Ausführungsform eines Körpers mit Spalt, in dem ein Spalt 600 zwischen einer Außenkante eines kugeligen Körpers 500, wie zum Beispiel einer Kugel und einer Bohrung hergestellt werden kann. Dazu ist die Kugel in einem Abstand 702 vor der Bohrung positioniert. Die Bohrung wirkt als Durchlass 300, der Abstand zwischen Außenkante und Bohrungskante bildet den Spalt 600. In Abhängigkeit des Abstands 702 variiert dessen Durchlassvolumen.

**Figur 3b** zeigt eine Einzelheit mit alternativer Ausführungsform eines Körpers mit Spalt, wobei ein Spalt 600 zwischen einem Stab 509 und einer Bohrung, den Durchlass 300 herstellt.

**Figur 3c** zeigt eine Einzelheit mit alternative Ausführungsform eines Körpers mit Spalt 600, der zwischen einem Keil 506 und einer Durchlassbohrung 300 hergestellt wird.

**Figur 3d** zeigt das Ausführungsbeispiel aus Figur 3c in einem Verschlusszustand. Die umlaufende Kante der Keilstirnfläche 507 eines Keils 506 dichtet gegen die Übergangskante 508 eines Durchlasses 300 zwischen trichterförmigem Einlass und schlauchartigen Abschnitt.

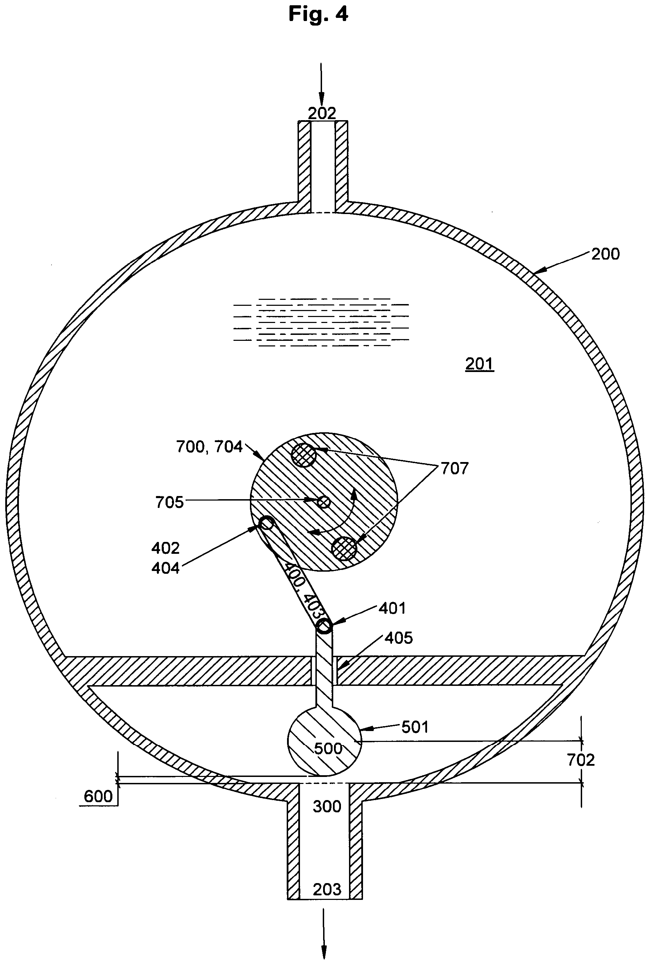

**Figur 4** zeigt eine alternative, zweite Ausführungsform der Erfindung in einer Seitenansicht. Danach sind im Gehäuseinnenraum 201 eines Gehäuses 200 mit Einlass 202 und Auslass 203 eine Kurvenscheibe 704 und ein Körper 500 mittels eines Kupplungselementes 400 verbunden, um eine Rotation bzw. eine Teilrotation der Kurvenscheibe 704 in eine Verkleinerung oder Vergrößerung des Abstands 702 zu übersetzen. Diese Veränderung entspricht einer Einstellung des Spalts 600 und damit einhergehend einer Einstellung des Durchlassvolumens. Dazu ist die Kurvenscheibe 704 auf einer Achse 705 gelagert.

Dazu ist ein Getriebeglied 403, zum Beispiel eine Getriebestange an mindestens zwei unterschiedlichen Enden jeweils an die Kurvenscheibe 704 und dem Körper 500 angeschlagen. In der vorliegenden Ausführungsform ist das Getriebeglied 403 in der Nähe des Außenrands der Kurvenscheibe 704 mittels eines Zapfens 404 an dessen zweiten Ende 402 an diese angeschlagen. Hingegen ist das andere Ende, das erste Ende 401 des Getriebeglieds 403, dass eine Durchlassbohrung aufweist in einem Maul des Körpers 500 gelagert.

Zur Führung des Körpers 500 ist dieser in einem Linearlager 405 gelagert.

Dadurch, dass in die Kurvenscheibe 704, eine Einstelleinheit 700 in der vorliegenden Ausführungsform mindestens ein Magnet 707 eingelassen ist, kann über eine magnetische Kupplung zwischen der Kurvenscheibe 704 und einem Einstellwerkzeug (nicht dargestellt) die Kurvenscheibe 704 rotiert, also eingestellt werden. Durch die Übersetzung dieser Rotation in einen definierten Abstand 702 zwischen Körper 500 und Durchlass 300 wird der Flusswiderstand, also die Größe des Spalts 600 bzw. dessen Durchlassvolumen einstellbar, sodass das Drainagevolumen des Hydrocephalusventils 100, also dem Sollausfluss eines Volumens an CSF einstellbar wird.

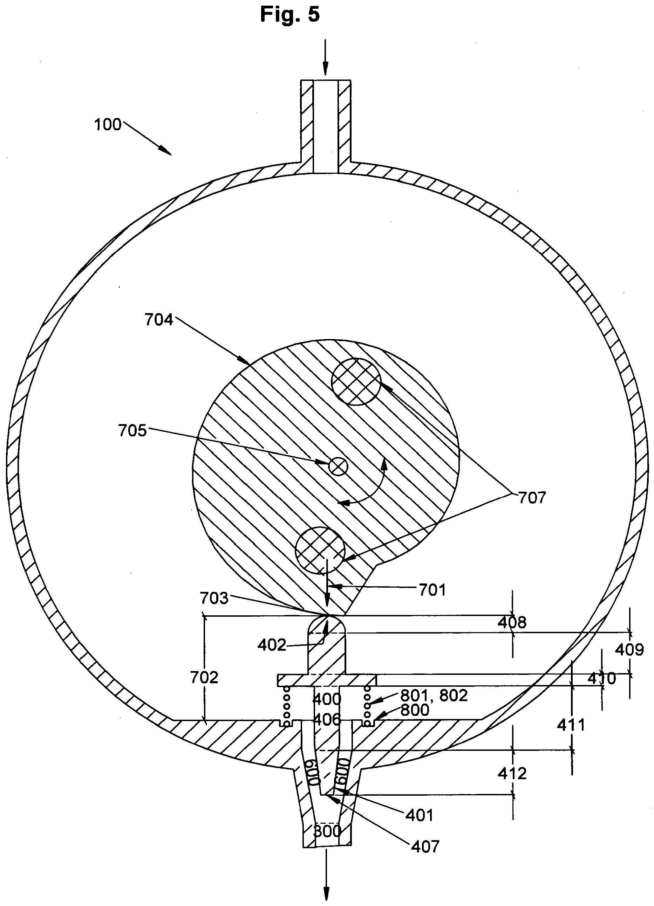

**Figur 5** zeigt eine alternative, dritte Ausführungsform der Erfindung in einer Seitenansicht. Dieser Ausführungsform nach umfasst das Hydrocephalusventil 100 eine Kurvenscheibe 704 mit einer Achse 705, einen Stecker 406, ein Linearlager 405, einer Feder 802 oder einem beliebigen anderen Federelement 801 und einen Durchlass 300. Die Feder 802 oder das Federelement sind dazu in einem Federsitz 800 gelagert. Dem Prinzip nach wird in dieser Ausführungsform eine Rotation der Kurvenscheibe 704 über einen Berührungspunkt 703 zwischen Kurvenscheibe 704 und Stecker 406 auf diesen in dessen Linearbewegung übersetzt. Der Stecker 406 wird bei seiner Bewegung durch die Feder gegen die Kurvenscheibe gedrückt. Um ein sicheres Hinein- und Heraus gleiten des Verschlussendes 407 des Steckers 406 in und aus dem Durchlass 300 bzw. dessen Durchlassendes zu gewährleisten, kann der Stecker 406 durch ein Linearlager 405 gelagert sein. In einer bevorzugten Ausführungsform erfolgt die Lagerung des Steckers 406 in einem seiner gleichförmigen Abschnitte. Dazu ist der Stecker 406 aus einem Materialblock, insbesondere Titanblock gedreht. Das Drehen vereinfacht ein Herstellen des Steckers 406 mit fünf Grundabschnitten, einem Berührabschnitt 408, einem Halsabschnitt 409, einem Kragenabschnitt 410, einem Verlängerungsabschnitt 411 und einem Verschlussabschnitt 412. Die Feder 802 ist in einem Federsitz 800 gelagert, der als Ring ausgestaltet ist. Durch die Bewegung des Stecker 406, z.B. in Form einer Nadel 1152 wird ein Spalt 600, bzw. ein Spaltdurchlass 1154 bzw. ein spaltähnlicher Durchlass erzeugt.

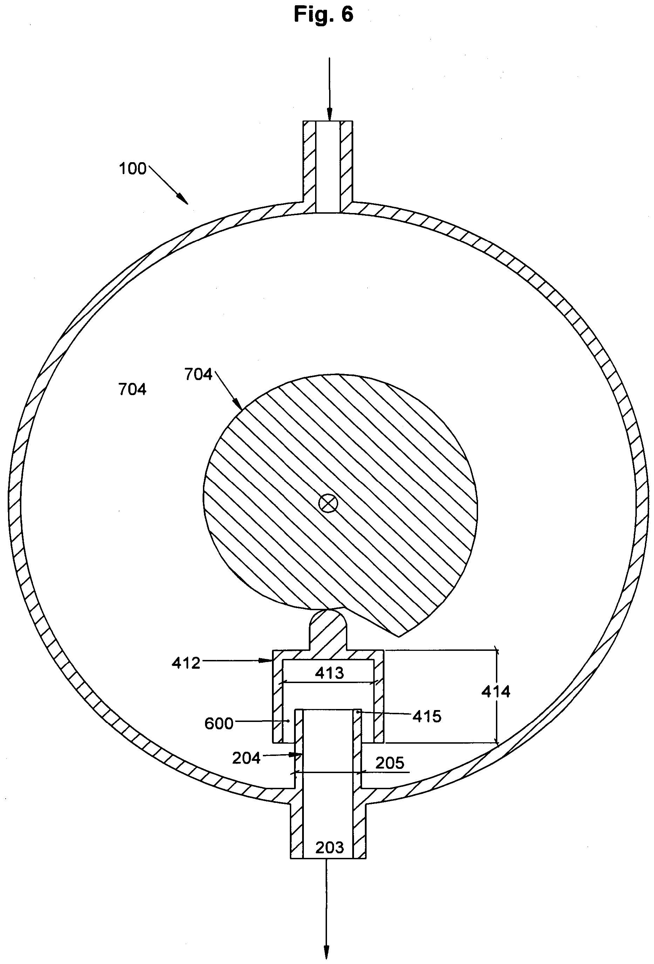

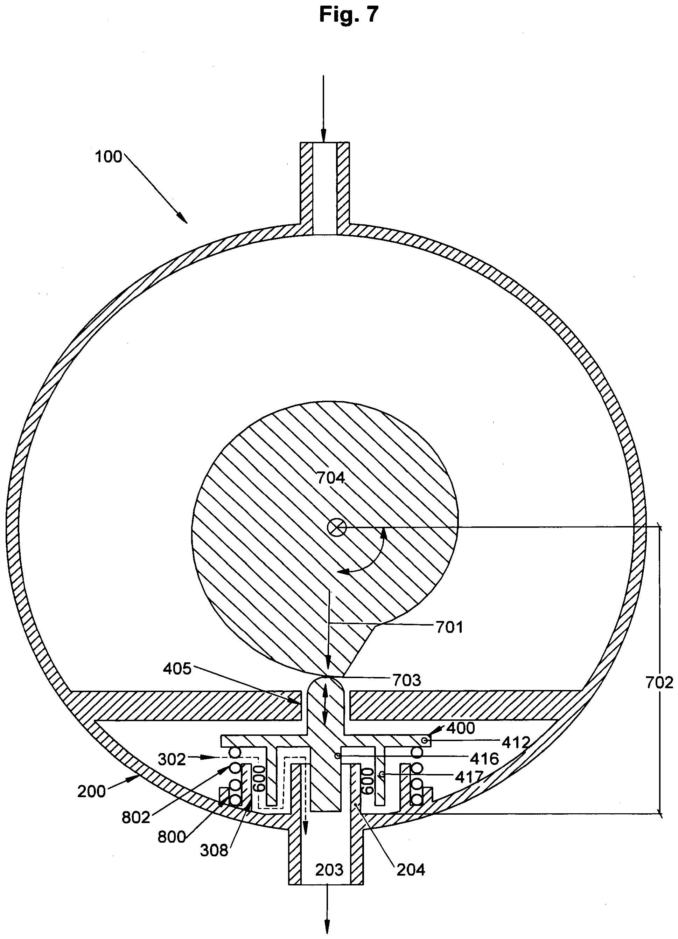

**Figur 6** zeigt eine alternative, vierte Ausführungsform der Erfindung in einer Ansicht von oben. Das dargestellte Hydrocephalusventil 100 umfasst dabei neben einer Kurvenscheibe 704 eine Hülse 412 und einen verlängerten Auslass 203 in Form eines Rohres 204. Das Rohr 204 kann ein Röhrchen, ein Schlauchstück, ein Schlauchende, ein Rohrabschnitt oder ein flexibler oder steifer Hohlkörper sein. In weiterer Ausgestaltung entspricht in etwa der Hülseninnendurchmesser 413 dem Rohraußendurchmesser 205. Die Entsprechung heißt für eine erste Ausführungsform: der Hülseninnendurchmesser 413 hat das gleiche Maß wie der Außendurchmesser 205 plus einem notwendigen Bewegungsspiel für eine Bewegung des Verschlußabschnittes 412 auf dem Rohr 204. In einer bevorzugten Ausführungsform weichen die Durchmesser weiter voneinander ab, so dass zwischen der Hülse 412 und dem Rohr 204 ein Spalt 600 entsteht. Das Rohr 204 läuft mit einem ersten Rohrende 415 in der Hülse 412. **Figur 7** zeigt ein erfindungsgemäßes Hydrocephalusventil 100 mit einem Kupplungselement 400 in teleskopartiger Ausgestaltung in einer Seitenansicht, das bevorzugt auf der Ausführungsform in **Figur 6** basiert. In der weiteren, teleskopartigen Ausgestaltung ist die Hülse 412 ebenfalls aus einem Titanblock gearbeitet, nämlich gefräst, allerdings sind ein Zentralstecker 416 und ein Rand 417 ausgefräst. Zusätzlich zu einem verlängerten Auslass 203 in Form eines Buchse 204 ist eine runde Auslassbuchse 308 koaxial zum Durchlass 203 im Gehäuse 200 eingelassen. Alternativ kann die Auslassbuchse 308 auch koaxial zum Durchlass 203 im Gehäuse 200, am Gehäuse 200 oder mittels Halteelementen innerhalb des Durchlasses 300 eingeklebt, eingesteckt, eingeschraubt, eingepresst oder eingeschmolzen werden. In weiterer Ausführungsform ist die Auslassbuchse 308 einstückig aus dem Gehäuse 200, einem Gehäusedeckel oder einem Gehäusetopf gefertigt. Supplementär ist die Hülse 412 in einem Linearlager 405 geführt. Die Spitze eines Hülsenendes berührt in einem Berührungspunkt 703 oder einer Berührungslinie oder einer Berührungsfläche die Außenkante der Kurvenscheibe 705. Eine Feder 802 ist in dieser Ausführungsform eine Druckfeder, sodass diese die Hülse 412 aus dem Auslass 203 herausdrückt, so dass ein Spalt 600 zwischen dem Zentralstecker 416 und dem Rohr 204 und zwischen dem Rand 417 und dem Rohr 204 und zwischen dem Rand 417 und der Auslassbuchse 308 resultiert. Der Abstand 702 zischen der Kurvenscheibe 704 und dem Ende des Kupplungselements beschreibt die Einstellbarkeit des Spalts 600, bzw. den Spaltdurchlass als einen möglichen Einstellparameter. Somit kann die Einstellbarkeit mathematisch beschrieben werden, indem der Zusammenhang zwischen Verdrehung der Kurvenscheibe 704 in ihrer Drehrichtung 701 und der Änderung des Abstands 702 dargestellt wird.

Die Feder 802 ist in einen Federsitz 800 gelagert.

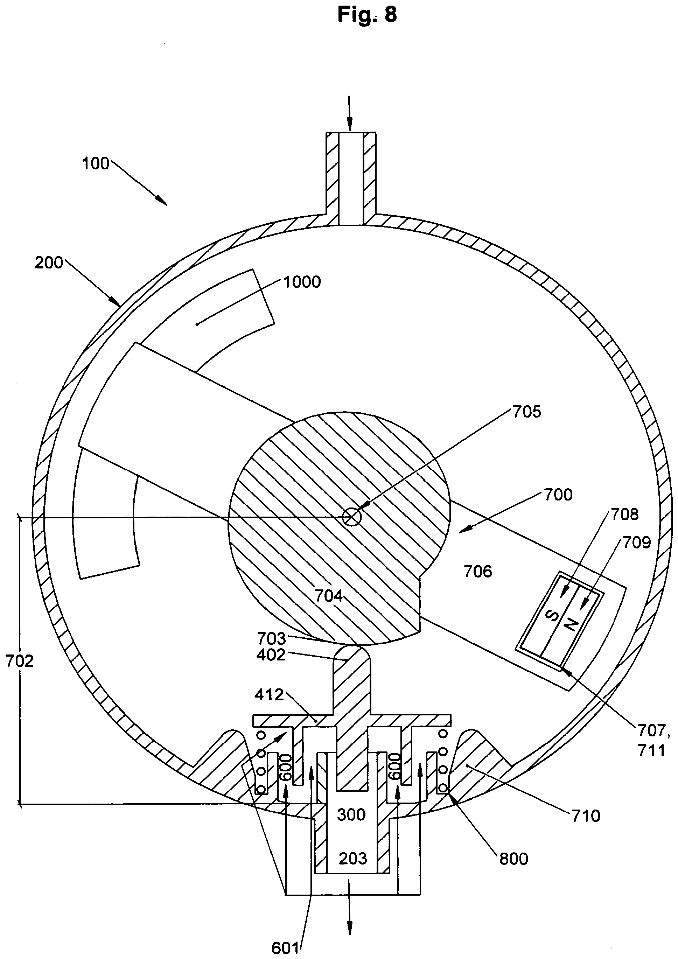

**Figur 8** zeigt das in **Figur 7** dargestellte erfindungsgemäße Hydrocephalusventil 100 mit einem Rotor 706 und mit magnetischen Kupplungsglied 711 mit Magneten 707, einem Nordpol 708 und einem Südpol 709. Die Einstelleinheit 700 umfasst mehrere Teile, darunter eine Kurvenscheibe 704 einen Rotor 706 und einen Schwenkarm 1050 oder einen Rotor 706 mit Schwenkarm 1050 oder eine Kurvenscheibe 704 mit Schwenkarm 1050. Die Kurvenscheibe 704 ist zusammen mit dem Rotor 706 auf der Achse 705 als eine Baugruppe gelagert. Der Rotor 706 umfasst einen Schwenkarm 1050 oder kann als Schwenkarm 1050 ausgearbeitet sein. Ferner sind die Kurvenscheibe 704 und der Rotor 706 miteinander verbunden. In bevorzugter Ausgestaltung sind sie miteinander verschraubt, verklebt, verschweißt oder aus einem Stück gefertigt. Die Verbindung zwischen Rotor 706 und Kurvenscheibe 704 resultiert in deren gemeinsamer Rotation/Schwenkbewegung, sodass eine Verdrehung des Rotors 706, bzw. des Schwenkarms 1050 oder eines zusätzlichen Schwenkarms 1050 einer proportionalen Verdrehung des Rotors 704 entspricht. Die Proportionalität folgt dabei dem Versatz der Kurvenscheibe 704 auf dem Rotor 706. In weiterer bevorzugter Ausgestaltung sind zwei Anschläge 710 in das Gehäuse 200 eingebracht, um einen Zusammenstoß zwischen Magneten 707 bzw. Rotor 706 und Feder 802 bzw. Hülse 412 zu verhindern. In der bevorzugten Ausführungsform kann die Größe des Spalts 600 zwischen Auslass 203 und Hülse 412 durch deren Hineinstecken in den Durchlass 300 oder deren Herausdrücken aus dem Durchlass 300 mittels einer Drehnung/Schwenkbewegung des Rotors 706 eingestellt werden. Dabei wird die Hülse 41 durch eine Feder 801, 802 aus dem Durchlass herausgedrückt. Die Feder ist in ihrem Federsitz 800 gehalten. Dieses kann durch eine Parametrisierung des Abstands 702 beschrieben werden.

In weiterer Ausgestaltung ist eine Bremse 1000 im Hydrocephalusventil 100 vorgesehen, um einen eingestellten Verdrehwinkel des Rotors 706 zu sichern. Dazu blockiert die Bremse 1000 die Rotationsfreiheit des Rotor 706 oder dessen Schwenkarms 1050. In einer bevorzugten Ausführungsform blockiert die Bremse 1000 den Rotor 706, in dem zwischen ihr und einer Rotorfläche ein Reibschluss zu- und abschaltbar ist. In einer alternativen Ausführungsform blockiert die Bremse 1000, in dem ein elektromagnetisches Kraftfeld den Rotor 706 in einer Wunschposition fixiert.

In einer unabhängigen Variante wird eine Mehrzahl unterschiedlicher Ventile miteinander kombiniert.

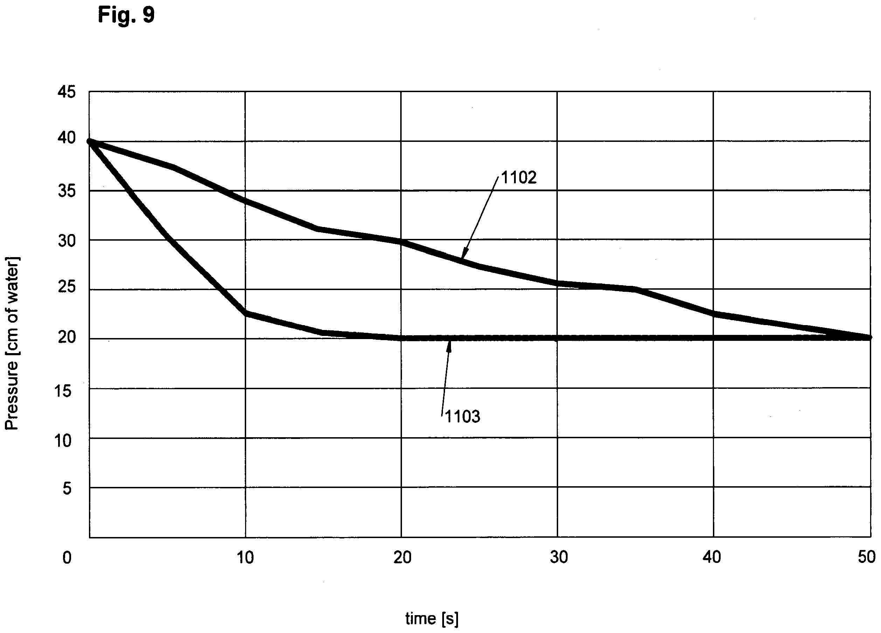

Figur 9 zeigt an einem herkömmlichen Hydrocephalusventil mit federbelasteter Kugel als Schließeinrichtung und mit üblicher Drainageleitung eine Aufnahme des Liquordruckes über der Zeit. Dabei hat sich eine Druckkurve 1103 ergeben. Die Druckkurve 1103 lässt erkennen, dass ein Liquordruck von 40 cm Wassersäule (entspricht 39,2266 mbar) innerhalb von rund 10 Sekunden auf ein Normalniveau von ca. 20 cm Wassersäule (entspricht 19,6133 mbar) abgebaut worden ist. Zum einen fühlt sich bei dieser Geschwindigkeit des Druckabbaus eine Vielzahl von Patienten unwohl. Zum anderen kann die Druckkurve für unterschiedliche Patienten mit unterschiedlichen Ventrikelsystemgrößen nur erreicht werden, wenn sie durch bauliche Parameter einstellbar ist. ist dies der Fall, ist sie patientenindividuell einstellbar, ist sie auf einen Sollverlauf einstellbar. Deshalb ist nach der Erfindung vorgesehen, einen Abfluss bzw. dessen Abflussgeschwindigkeit einstellbar zu machen, um einen Druckabbau zu realisieren, welcher auf eine längere Zeit, vorzugsweise auf mindestens 20 Sekunden, noch weiter bevorzugt auf mindestens 30 Sekunden und höchstbevorzugt auf mindestens 40 Sekunden patientenunabhängig eine gleiche Wirkung auf diesen hervorbringt. Vorzugsweise verteilt sich der Druckabbau maximal auf 60 Sekunden, Noch weiter bevorzugt auf maximal 50 Sekunden.

Die Druckkurve 1103 der Fig. 9 zeigt einen Verlauf der Druckkurve über 50 Sekunden. Die über der Zeit (Abszisse) in Sekunden aufgetragene Druckkurve 1103 zeigt eine Neigung zur Abszisse, die in Abhängigkeit von dem Maßstab für die Druckwerte (Ordinate) und zum Maßstab für die Sekunden (Abszisse) steht. Alles, was bei gleichen Maßstäben für Druckwerte und Sekunden eine gleiche oder geringere Neigung zur Abszisse hat, liegt im Rahmen der Erfindung, vorausgesetzt die Neigung ist nicht geringer als bei einer Verteilung des Druckabbaus auf 60 Sekunden, vorzugsweise nicht geringer als bei einer Verteilung des Druckabbaus auf 50 Sekunden.

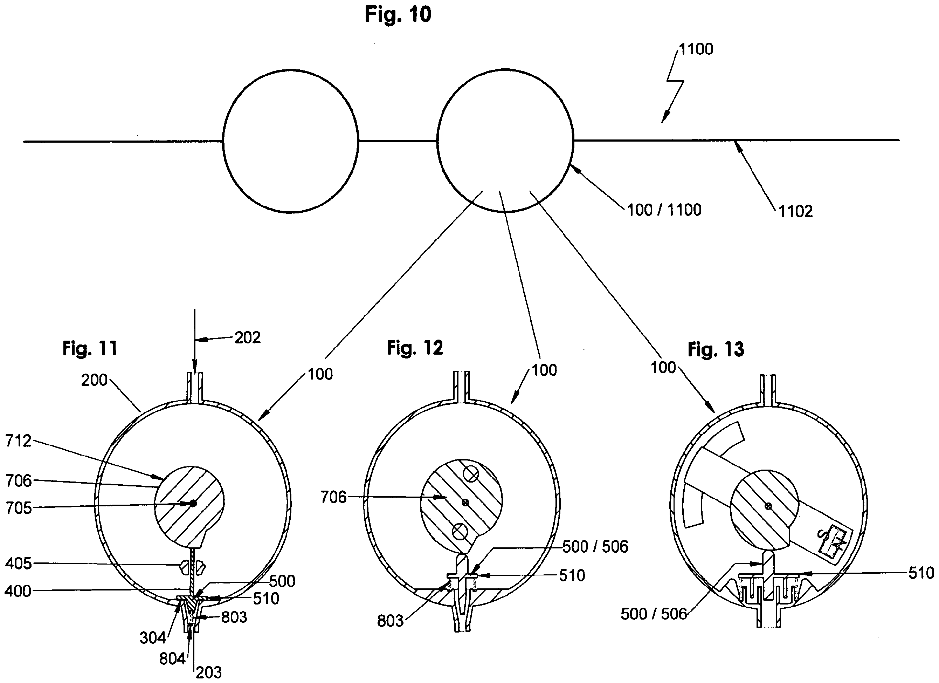

Fig. 10 zeigt eine bevorzugte Ausführungsform einer Variante des erfinderischen Hydrocephalusventils als Ventilkombination 1100.

Die nachfolgend erläuterten einstellbaren Ventilkombinationen sind bei elektronischer Steuerung zusammen mit einer nicht dargestellten Druckmessung ohne weitere Hilfsmaßnahmen in der Lage, eine gewünschte Druckkurve 1103 **(****Fig.9****)** zu fahren. Sie sind in Kombination, auch in Kombination mit herkömmlichen Hydrocephalusventilen auch in der Lage auf rein mechanischer Basis zumindest annähernd eine gewünschte Druckkurve 1103 zu fahren. Die Ventilkombination ist schematisch in **Fig. 10** dargestellt. Nach **Fig. 10** umfasst sie eine Drainageleitung 1102. In der Drainageleitung 1102 sind ein herkömmliches Hydrocephalusventil und eines der nachfolgend erfinderischen Hydrocephalusventile 100 eingebaut.

Alternativ zeigt **Fig. 10** die Erfindung als eine Verschaltung aus einem herkömmlichen Ventil mit einer erfinderischen Ventilkombination 1100, also eine erweitere Ventilkombination.

**Figur 11** zeigt ein erfindungsgemäßes Hydrocephalusventil 100 in einer Schnittansicht von oben. **Fig. 11** ist eine Verkleinerung von **Fig. 1****.** Zu dem Hydrocephalusventil 100 gehört ein flüssigkeitsdichtes Gehäuse 200, das mit einem Einlass 202 und einem Auslass 203 versehen ist. In dem Gehäuse 200 ist eine Achse 705 angeordnet. Auf der Achse sitzt ein Rotor 706. Der Rotor 706 bildet eine Kurvenscheibe 704 mit einer ersten Kurvenbahn 712. Auf der ersten Kurvenbahn 712 gleitet ein Kupplungselement, ein Stift 400 mit seinem zweiten Ende 402. Der Stift 400 ist zwischen Führungen 405 des Gehäuses 200 in radialer Richtung zu der Achse 705 beweglich gehalten. An dem gegenüber liegenden ersten Ende 401 des Stiftes 400 ist ein Körper 500 in Form eines Stopfens vorgesehen. Der Körper 500 besitzt eine kegelige Spitze und einen, Kragen 510. Nach **Fig. 11** kann der Kragen 510 mit einer Dichtfläche 304 des Gehäuses 200 dicht abschließen. Das verhindert einen unerwünschten Austritt von Liquor. Dabei wird der Stopfen von der Kurvenbahn 712 in radialer Richtung nach außen gedrückt. Bei Verschwenken des Rotors 706 entgegen dem Uhrzeigersinn in **Fig**. **11** reduziert sich der Radius der Kurvenbahn 712. Dabei bleibt der Stift 400 in Berührung mit der Kurvenbahn 712. Das erfolgt unter dem Druck einer Spiralfeder 803. Die Spiralfeder 803 sitzt mit einem Ende in einer Ausnehmung der kegeligen Spitze. Die Spiralfeder 803 stützt sich am anderen Ende an einem Steg 804 des Auslasses 203 ab. Wenn der Stift 400 unter dem Druck der Spiralfeder 802 in radialer Richtung in das Gehäuse 200 bewegt wird, bildet sich zwischen dem Stopfen und der Dichtfläche am Gehäuse ein Durchlass für Liquor. Im Ausführungsbeispiel nach **Fig. 1** sitzt die Kurvenscheibe 706 fest auf der Achse und die Achse ist mit einem nicht dargestellten elektrischen Schrittschaltmotor verbunden. Der Schrittschaltmotor wird von einer speicherbaren Steuerung in Gang gesetzt. In der Steuerung ist ein in **Fig. 9** gewünschter zeitlicher Verlauf des Druckabfalls in dem Liquor gespeichert. Dieser Verlauf wird in der Steuerung mit Hilfe eines Algorithmus mit den Druckwerten einer nicht dargestellten Druckmesseinrichtung verglichen. Die Differenz zwischen beiden Werten führt zu einem Steuerungsimpuls an den elektrischen Schrittschaltmotor.

**Figur 12** ist eine verkleinerte Darstellung von **Fig. 5****.** Das Ausführungsbeispiel nach **Fig. 12** unterscheidet sich von dem Ausführungsbeispiel nach **Fig. 11** dadurch, dass der Körper 500 ein Keil 506 ist, bzw. ein keilförmiger Stopfen ist, der in einen passenden Abfluss ragt. Im Ausführungsbeispiel heißt das, der Abfluss umgibt den keilförmigen Stopfen in allen Stellungen im Abstand. Der Abstand ergibt sich entsprechend der Abmessung des Stopfens an der Stirnfläche und abhängig von der jeweiligen Stellung des keilförmigen Stopfens. Keile 506 besitzen einen rechteckigen Querschnitt mit zwei zueinander geneigte Seiten und zwei zueinander parallelen Seiten. Der keilförmige Stopfen ist wie der Stopfen nach **Fig. 11** mit einem, hier nicht dargestellten Stift gehalten. Auch in der Ausführungsform nach **Fig. 12** ist eine Spiralfeder 803 vorgesehen, die eine bleibende Berührung des Stiftes mit der Kurvenscheibe sicherstellt. Anders als nach **Fig. 1** sitzt die Spiralfeder 803 nicht auf der Spitze des Stopfens, sondern auf dem Stift, wobei ein Kragen 510 auf dem Stift sitzt. Die Feder befindet sich zwischen dem Kragen und der Gehäuseinnenwand, wobei die Feder den Stift und den Stopfen umgibt. Der keilförmige Stopfen verfügt über einen anderen Dreh- und Schwenkantrieb, nämlich einen Rotor 706, wie ein herkömmliches Hydrocephalusventil, zusammen mit einer herkömmlichen Arretierung zwischen zwei Verstellvorgängen. Wie oben erläutert wird der Rotor besonders häufig mit Magneten bewegt, von denen ein Teil in dem Rotor verbaut ist und der andere Teil in einer Verstelleinrichtung angeordnet ist, die über dem implantierten Ventil auf der Haut des Patienten aufgesetzt wird und durch Drücken die Arretierung löst und durch Drehen den Rotor verschwenkt. Das so entstandene Ventil wird nach **Fig. 10** mit einem herkömmlichen Hydrocephalusventil kombiniert, **Fig. 10 und Fig 12** zeigen eine Drainageleitung 1101 **(****Fig.10****)** mit einem erfinderischen Hydrocephalusventil 100, das zur Einstellung einer Abflussgeschwindigkeit bestimmt ist und einem herkömmlichen Hydrocephalusventil in der Drainageleitung 1101 nachgeschaltet ist.

**Figur 13** ist eine verkleinerte Darstellung von **Fig. 8**. **Fig. 13** zeigt eine Situation, in der die Gehäusewand einen Abstand von dem Schwenkarm 1050 hat. Der Abstand entsteht, wenn mit der Verstellvorrichtung zur Verstellung der Kurvenscheibe oben auf das implantierte Gehäuse gedrückt wird. Dadurch verformt sich das Gehäuse, so dass der vorher an Gehäuseinnenwand reibungsschlüssig anliegende Schwenkarm 1050 zur Verstellung frei gegeben wird. Eine erfolgreiche Freigabe wird durch einen Klicklaut signalisiert, weil der obere Teil des Gehäuses als Knackmembran, also eine gestufte Rundmembran (nicht dargestellt) ausgeführt ist. Bevorzugterweise sind dazu in die Rundmembran (nicht dargestellt) eine Mehrzahl an Stufen gefertigt. Der Körper 500 mit Kragen 510 ist als gestufter Deckel ausgeführt.

**Figur 11**, **Fig. 12** und **Fig. 13** zeigen gleichfalls einen Körper 500, der in jeder Stellung zu dem umgebenden Einlass oder Auslass einen Abstand aufweist, In der Ausführung nach **Fig. 12** und **Fig. 13** steht der Körper mittelbar mit einer Kurvenscheibe 704 in Berührung. Im Unterschied zu **Fig. 11** erfolgt die Berührung mit einer verdickten Verlängerung des Körpers 500. Am Übergang des Körpers 500 zu seiner Verlängerung ist der erläutere Kragen 510 dargestellt. Zwischen dem Kragen 510 und der Gehäuseinnenwand ist die mit 802 bezeichnete Feder angeordnet. Die Feder 802 umgibt einen Körper 500, hier einen Stopfen und greift dabei in eine zentrierende Nut. Außerdem sind in Kurvenscheibe 704 zwei beschriebene Magnete eingelassen (**Fig. 12**).

Neben den vorgenannten Kombinationen mit Ventilen aus den Figuren **Fig. 1****,** **Fig. 5** und **Fig. 8** entstehen weitere vorteilhafte Kombinationen mit den Ausführungsformen von **Fig. 4****,** **Fig, 6** **und** **Fig. 7****.**

Eine Kombination (nicht dargestellt) mit der Ausführungsform aus **Fig. 4** resultiert in einem Körper, der als kugelförmiger Stopfen ausgebildet ist. Der Durchmesser des kugelförmigen Stopfens ist größer als die Öffnungsweite des Einlasses oder Auslasses, so dass der kugelförmige Stopfen sowohl ein Ventil mit auf-zu-Funktion als auch eine Einstellung der Öffnungsweite vornehmen kann. Dabei ist der kugelförmige Stopfen mit einer Stange in einer Führung verschiebbar gehalten. Außerdem ist der kugelförmigen Stopfen über eine Gelenkstange gelenkig mit einem schwenkbeweglichen Rotor in dem Ventilgehäuse gehalten. Im Ausführungsbeispiel sind für die Verstellung des kugelförmigen Stopfens Magnete im Rotor und eine Verstellvorrichtung vorgesehen, die gleichfalls Magnete aufweist und über dem implantierten Ventil auf der Haut des Patienten aufgesetzt und von Hand gedreht/verschwenkt wird. Anstelle der Verstellvorrichtung kann auch ein speicherbarer Schrittschaltmotor verwendet werden. Mit einem Schrittschaltmotor kann der kugelförmigen Stopfen in jede gewünschte Stellunggebracht werden.

Eine Kombination (nicht dargestellt) mit der Ausführungsform aus **Fig. 6** lässt ein weiteres Ventil mit einem als topfartigen Stopfen ausgebildeten Körper entstehen, wobei der topfartige Stopfen über einem in das Ventilgehäuse vorragenden Auslass sitzt. Auch der topfartige Stopfen wird mit einer Kurvenscheibe verstellt. Dabei wird der topfartige Stopfen wie der Stopfen nach **Fig. 6** in nicht dargestellter Form in dem Ventilgehäuse geführt und mit einer Feder mit der Kurvenscheibe in Berührung gehalten. Dieser Stopfen bietet die gleichen Anwendungsmöglichkeiten wie der zylindrische Stopfen nach **Fig. 3b**. Während nach **Fig. 3b** der bleibende Spalt zwischen dem zylindrischen Stopfen und dem umgebenden, rohrförmigen Einlass oder Auslass den Grenzwert für die Liquorströmung bilden, ist im Ausführungsbeispiel der bleibende Spalt (Grenzwert) zwischen dem rohrförmigen Auslass oder Einlass und dem Innenmantel des umgebenden topförmigen Stopfens maßgebend. Wie bei dem Stopfen nach **Fig. 3b** bleiben Öffnungsbewegungen des Stopfens auch bei dem Ausführungsbeispiel ohne Einfluss, wenn sie den Grenzwert für den Öffnungsquerschnitt überschreiten.

Eine Kombination (nicht dargestellt) mit der Ausführungsform aus Fig. 7 zeigt ein Ventil, das auch einen zylindrischen Stopfen umfasst. Zu dem zylindrischen Stopfen gehört ein angepasster rohrförmiger Auslass. Der rohrförmige Auslass umgibt den zylindrischen Stopfen in einem gewählten Abstand. Der zylindrischen Stopfen besitzt einen Kragen und in radialer Richtung zu einer Kurvenscheibe eine verdickte Verlängerung. Die Verlängerung berührt die Kurvenscheibe. An der Berührungsstelle ist die Verlängerung gerundet. Der zylindrische Stopfen ist an der Verlängerung in einer Führung des Gehäuses in radialer Richtung verschiebbar gehalten. Mit einer Spiralfeder zwischen dem Kragen und der Innenwand des Gehäuses wird sichergestellt, dass der Stopfen immer in Berührung mit der Kurvenscheibe bleibt. Durch Verschenken der Kurvenscheibe wird der Öffnungspalt zwischen dem Kragen und dem Auslass eingestellt. Bei einer Reduzierung des Öffnungsspaltes drückt die Kurvenscheibe den Kragen nach außen. Bei einer Vergrößerung des Öffnungsspaltes gibt die Kurvenscheibe Raum, so dass der Stopfen der Kurvenbahn der Kurvenscheibe unter dem Druck der Spiralfeder folgt. Das Verschwenken der Kurvenscheibe erfolgt im Ausführungsbeispiel mit Magneten, wobei sich Magnete sowohl in der Kurvenscheibe als auch in einer Verstellvorrichtung befinden, die zum Verstellen über dem implantierten Ventil auf der Haut des Patienten aufgesetzt und gedreht wird. Im Ausführungsbeispiel sind konzentrisch zu dem zylindrischen Stopfen und dem Auslass noch ein ringförmiger Steg am Kragen und ein ringförmiger Steg an der Innenwand des Gehäuses vorgesehen. Diese Stege zwingen den Liquor zu einem mäandernden Strömungsverlauf. Das Ausführungsbeispiel unterscheidet sich durch einen Schwenkarm an der Kurvenscheibe. Der Schwenkarm dient der Aufnahme der Magnete. Außerdem ist eine andere Führung des Stopfens in dem Ventilgehäuse vorgesehen.

Eine bevorzugte Form des vorgenannten Körpers hat die Form einer Nadel. **Fig. 2a, 2b**, **3a und 3b** zeigen unterschiedliche Körper. Bis auf **Fig. 2b** handelt es sich um konische Stopfen für ein Ventil, wobei der Stopfen eine Spitze aufweist, die dicker als die Öffnung des Einlasses oder Auslasses für den Liquor ist. Der konische Stopfen kann auch als eine Nadel aufgefasst werden. Die Ausführungsform als Nadel ist Teil jeder vorgenannten Ausführungsform, z.B. nach **Fig. 11**, **Fig. 12** und **Fig. 14****.**

Auf Grund der Form einer Nadel verschließt der Körper, bzw. der Stopfen den Einlass oder Auslass, wenn die Nadel weit genug in den Einlass oder Auslass gedrückt wird. **Fig. 3c** zeigt die Offenstellung des Ventils, **Fig. 3d** die Schließstellung. Das zu dem Stopfen nach **Fig. 3c und 3d** gehörige Ventil entspricht im Übrigen dem Ventil nach **Fig. 2a. Fig. 2b** zeigt einen genau zylindrischen Stopfen anstelle konischer oder keilförmiger Stopfen. Zu dem zylindrischen Stopfen gehört ein passender rohrförmiger Einlass oder Auslass an dem Ventil, der den Stopfen im Abstand umgibt. Gleichgültig, wie weit der zylindrische Stopfen in den Einlass oder Auslass bewegt wird, der Abstand zwischen dem Stopfen und dem umgebenden Einlass oder Auslass bleibt unverändert. Der zylindrische Stopfen eignet sich für ein Ventil, dass im Übrigen die Merkmale des zur **Fig**. **2a** gehörigen Ventils besitzt. Der zylindrische Stopfen eignet sich aber auch für eine Drainage mit einem einzigen Ventil in der Drainageleitung und gleichzeitiger, oben beschriebener Reduzierung des Druckabfalls. Dabei treten der zylindrische Stopfen und der passende rohrförmige Einlass oder Auslass an die Stelle der üblichen Kugel und des üblichen Ventilsitzes für die Kugel. Außerdem an dem zylindrischen Stopfen noch ein Kragen wie nach **Fig. 1** vorgesehen. Der Kragen hat hier eine Schließfunktion. Der Kragen legt sich schließend an die Innenwand des Ventilgehäuses. Auf dem zylindrischen Stopfen lastet eine Feder, die den Öffnungsdruck des Ventils bestimmt. Die Feder ist in herkömmlicher Weise verstellbar, so dass auch der Öffnungsdruck verstellbar ist. Je höher der Liquordruck ansteigt, desto weiter entfernt sich der Kragen von der Schließfläche der Innenwand des Ventilgehäuses. Das führt jedoch nur bis zu einer Grenze auch zu einer größeren Öffnungsweite des Ventils. Die Öffnungsweite kann nicht größer werden als der Spalt zwischen dem Stopfen und dem umgebenden Einlass oder Auslass. Je größer die von dem Liquordruck versachte Strömungsgeschwindigkeit des Liquors durch das Ventilwird, desto größer wird der Strömungswiderstand. Das bewirkt eine Verlangsamung der Zunahme der Strömungsgeschwindigkeit und des Druckabfalls.

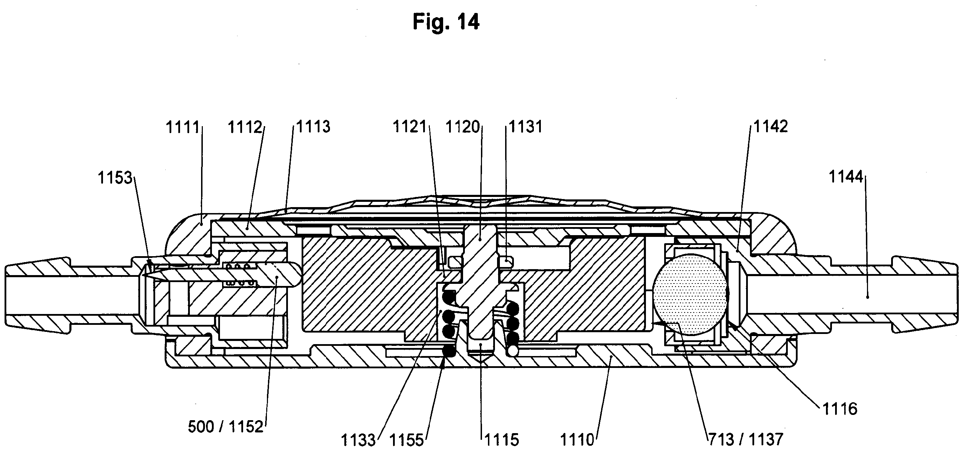

Fig. 14 bis 18 zeigen eine bevorzugte Ausführungsform der Erfindung. Diese Ausführungsform kann auch unabhängig ausgeführt werden.

**Figur 14** zeigt einen Schnitt durch eine erfindungsgemäße Vorrichtung 100 mit zwei Ventilen in einem gemeinsamen Gehäuse, eine Ventilkombination 1100. Das Gehäuse mit den beiden Ventilen ist Bestandteil eine Liquor-Drainage bzw. eines Shunts eines Hydrocephaluspatienten. Die Ventilkombination 1100 ist dazu mit entsprechenden Leitungen (nicht dargestellt), sog. Kathetern unter der Haut des Patienten implantiert. Die Leitungen führen den Liquor aus dem Schädel (nicht dargestellt) des Patienten zu der erfindungsgemäßen Vorrichtung und von dort aus in den Bauchraum (nicht dargestellt) des Patienten, wo der Körper des Patienten den Liquor absorbiert. Das Gehäuse besteht aus einem Boden 1110, einem Ring 1111 einer ringförmigen Stützscheibe 1112 und einem Deckel 1113. Mittig im Gehäuse sitzt eine Achse 1120 verschiebbar. Dabei ist die Achse 1120 an einem Ende in der Stützscheibe 1112 und am anderen Ende in einer Führung 1114 des Bodens 1110 gehalten. Die Achse 1120 besitzt einen Außenkragen 1121 und stützt sich mit dem Außenkragen 1121 über eine Spiralfeder 1122 an dem Boden 1110 ab. Auf der Achse 1120 sitzen ferner ein Rotor 1130 und ein Sicherungsring 1131. Dabei greift der Rotor 1130 mit einem Innenkragen 1132 zwischen den Außenkragen 1121 und den Sicherungsring 1131. Außerdem umgibt der Rotor 1130 die Spiralfeder 1122. Dazu ist in dem Rotor 1130 eine entsprechende Ausnehmung 1133 vorgesehen. Die Spiralfeder 1122 drückt den Rotor 1130 gegen die Stützscheibe 1112 im Gehäuse, so dass Reibungsschluß zwischen dem Rotor 1130 und der Stützscheibe 1112 besteht und der Rotor 1130 in der betreffenden Stellung arretiert wird. In dieser Stellung hat der Deckel 1113 eine nach außen gerichtete Wölbung. Die Arretierung kann gelöst werden, in dem mit einer Verstellvorrichtung, die über dem Gehäuse auf der Haut der Patienten aufgesetzt gegen das Gehäuse gedrückt wird. Alternativ kann die Bremse auch gelöst werden, wenn manuell der Deckel 1113 gedrückt wird.

Der Druck führt zu einer Einbeulung des Deckels 1113 und zu einer Verschiebung der Achse 1120 gegen den Boden 1110. Dabei kann die Achse 1120 sich in den Hohlraum 1115 der Führung 1114 verschieben. Schon eine geringe Verschiebung der Achse 1120 führt zu einem Lösen des Rotors 1130 von der Stützscheibe 1112. Danach kann der Rotor 1130 verschwenkt werden. Zum Verschwenken des Rotors 1130 sind in dem Rotor 1130 Magnete 1134 an diametral gegenüberliegenden Stellen verbaut.

**Figur 14** zeigt eine Situation einer Erfindung 100, 1100 mit einer Kugel 1141 bei abgeschaltetem Ventil. Dabei wird die Kugel 1141 von einer Tellerfeder 1147 einen Ventilsitz gedrückt. **Fig**. **14** zeigt einen zweiten Einsatz 1151. In dem Einsatz 1151 ist ein Körper 500, 1152 in Form eines zylindrischen Schließteils 1152, z.B. in Form einer Nadel 1152 verschiebbar angeordnet. Die Nadel 1152 ragt mit einer konischen Spitze 1153 in den Spaltdurchlass 1154, einen kleinen Kanal, einen Spalt. Die Stellung der Spitze 1153 bestimmt die Öffnungsweite des Spaltdurchlasses 1154 für den Liquordurchtritt. Je weitere die Spitze 1153 in den Spaltdurchlass 1154 reicht, desto kleiner wird die Öffnungsweite für den Durchtritt von Liquor in den Auslass 1150. Je weniger die Spitze 1153 in den Spaltdurchlass 1154 reicht, desto größer wird die Öffnungsweite für den Durchtritt von Liquor.

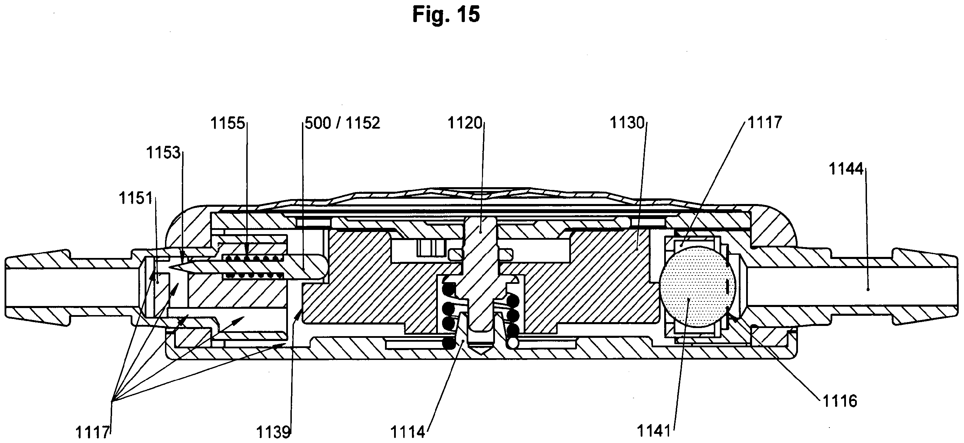

Die Stellung der Spitze 1153 bzw. des Schließteiles 1152 wird gleichfalls durch eine zweite Kurvenbahn 714 an dem Rotor 1130 bestimmt. Nach **Fig. 14** **und** **15** verläuft die Kurvenbahn 714 für das Schließteil 1152, einen Körper 500, also die Nadel 1152 oberhalb der ersten Kurvenbahn 712 für die Kugel 1141 für das Differenzdruckventil 1140b oder ein Gravitationsventil 1140a (nicht dargestellt). Die zweite Kurvenbahnfläche 715 wird durch die Berührungsfläche der Nadel 1152 mit dem Rotor 1130 gebildet. Die erste Kurvenbahnfläche 713 wird durch die Berührungsfläche der Kugel 1141 und der ersten Kurvenbahn 712 gebildet. Der Rotor 1130 ist auf einer Achse 1120 gelagert. Ein Außenkragen 1121 der Achse 1120 stützt den Innenkragen 1132 des Rotors 1130 und wird von einem Sicherungsring 1131 gehalten. Dafür sitzt die Achse 1120 in einer Ausnehmung 1133 des Rotors 1130.

Wie bei der Kugel 1141 ist ständig eine Berührung des Schließteiles 1152, der Nadel 1152 mit dem Rotor 1130 vorgesehen, so dass die Nadel 1152 ständig der für ihn bestimmten Kurvenbahn 715 folgt. Dazu ist die Nadel 1152 von einer Spiralfeder 1155 umgeben, die die Nadel 1152 gegen den Rotor drückt. Die Spiralfeder 1155 stützt sich dazu mit dem einen Ende in dem Einsatz 1151 ab. Mit dem anderen Ende drückt die Spiralfeder gegen die Nadel 1152.

**Figur 15** zeigt die Erfindung gemäß **Fig. 14** mit gleicher Schnittdarstellung aber in anderer Schwenkstellung des Rotors 1130, in einem geöffneten Zustand. Die Spiralfeder 1155 drückt die Nadel 500, 152 aus dem Spaltdurchlass 1154 heraus, so dass die Kanäle 1117 von Liquor durchflossen werden können. Ferner befinden sich passende Magnete 1134 in der Verstellvorrichtung, so dass eine Drehung der Verstellvorrichtung um die Achse 1120 in ihrer Führung 1114über die Anziehungskraft der Magnete 1134 ein Verschwenken des Rotors bewirkt. Wenn nach einem gewünschten Verschwenken die Verstellvorrichtung wieder entfernt wird, erfolgt eine neuerliche automatische Arretierung des Rotors. Durch Verschwenken des Rotors 1130 kann ein Gravitationsventil 1140a (nicht dargestellt) oder ein Differenzdruckventil 1140b in dem Gehäuse zugeschaltet oder abgeschaltet werden.

Das Gravitationsventil 1140 oder das Differenzdruckventil 1140b ist einlassseitig in dem Gehäuse angeordnet. Die Strömungsrichtung ist mit 1146 bezeichnet. Zu dem Gravitationsventil 1140a oder dem Differenzdruckventil 1140b gehört eine Kugel 1141 Die Kugel 1141 sitzt in einem Einsatz 1142 in ihrem Ventilsitz 1116. Der Einsatz 1142 ragt mit einer Anschlußtülle 1143 durch eine Öffnung in dem Ring 1111 des Gehäuses. Die Anschlußtülle 1143 dient dem Anschluß einer Schlauchleitung.

Die **Fig. 15** zeigt das Differenzdruckventil 1140b offen in einer Liegendstellung des Patienten. Der Rotor 1130 drückt mit einer ersten Berührungsfläche, erste Kurvenbahnfläche 713 gegen die Außenwand der Kugel, so dass diese gegen die Federkraft einer Tellerfeder 1147 in den ersten Einsatz 1142 in Richtung Öffnung 1144 versetzt wird. Durch diesen Versatz werden Kanäle 1117 frei, so dass einströmender Liquor passieren kann. Weil durch das Gehäuse Kanäle 1117 führen, kann der Liquor die Erfindung durchströmen.

Für den Fall, dass ein Gravitationsventil 1140a verbaut ist und der Patient mit der in **Fig. 15** dargestellten Ventilstellung eine Stehendlage einnimmt, schließt das Gravitationsventil 1140a unter dem Druck der Kugel 1141.Unabhängig davon kann das Gravitationsventil 1140 abgeschaltet werden, so dass immer, auch in der Liegendlage des Patienten eine Liquorströmung verhindert wird.

Um von der in **Fig. 14** dargestellten Kurvenbahnfläche - geschlossen - zu der in **Fig. 15** dargestellten Kurvenbahnfläche - geöffnet - zu kommen, muss der Rotor 1130 um ein bestimmtes Maß verschenkt werden. Das geschieht in der oben beschriebenen Weise mit der Verstellvorrichtung. Auch in einem anderen, auslassseitig angeordneten Einsatz 1151 befinden sich Kanäle 1117.

**Die** **Figuren 14** **und** **15** zeigen unterschiedliche Stellungen des Schließteiles 1152. Nach **Fig. 14** ist die Spitze 1153 weit in den Spaltdurchlass 1154 eingedrungen. Die zweite Kurvenbahn 714 für das Schließteil 1152 mit dem Rotor 1130 verläuft in **Fig. 14** an der Berührungsfläche in einem für die Stellung der Spitze 1153 ausreichend großem Abstand von der Achse 1120. Nach **Fig. 15** ist der Abstand der Berührungsfläche von der Achse 1120 wesentlich geringer, so dass die Spitze 1153 um dieses Maß gegenüber der Stellung nach **Fig. 14** zurückgesetzt ist und sich eine größere Öffnungsweite zwischen der Spitze 1153 und dem Spaltdurchlass 1154 ergibt. Daher zeigt **Fig. 15** die Erfindung in einem Öffnungszustand.

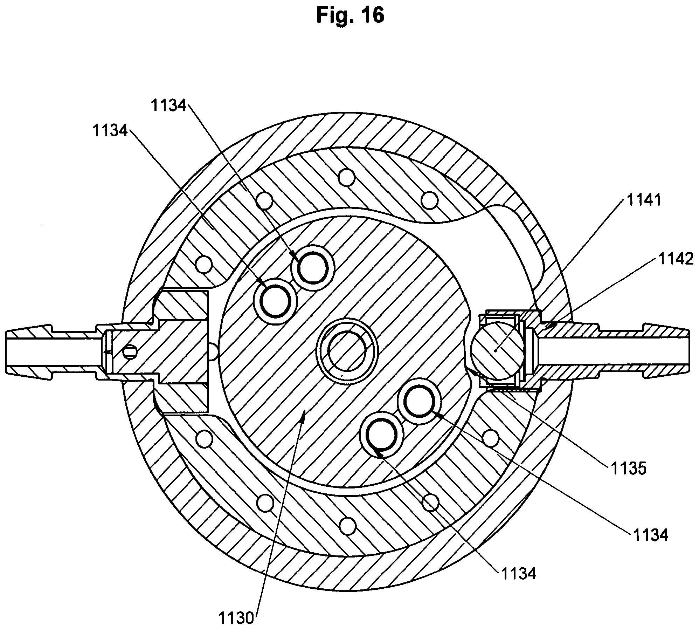

**Figur 16** zeigt einen Schnitt durch die erfindungsgemäße Vorrichtung, wobei die Mittelachsen der Einsätze 1142 und 1151 in der Schnittebene liegen.

Das Schließteil 1152, die Nadel 1152 ist in der Schnittdarstellung bedingt ersichtlich, weil sie einen Abstand von der Schnittebene hat. Die Kugel 1141 ist in der Schließstellung dargestellt.

Der Rotor 1130 besitzt eine Einwölbung 1135 und eine Auswölbung 1136. Im Bereich der Einwölbung 1136 befindet sich die Kugel 1141 in der Schließstellung. Das Gravitations- 1140a, oder Differenzdruckventil 1140b ist abgeschaltet. Im Bereich der Auswölbung 1135 ist das Gravitationsventil wieder zugeschaltet. Bei stufenweiser Zuschaltung sind zwischen der Einwölbung 1135 und der Auswölbung 1136 noch weniger tiefe Einwölbungen und/oder weniger starke Auswölbungen vorgesehen. **Fig. 16** zeigt im Ausführungsbeispiel vier Magnete 1134 für den Rotor 1130 zu dessen Verstellung.

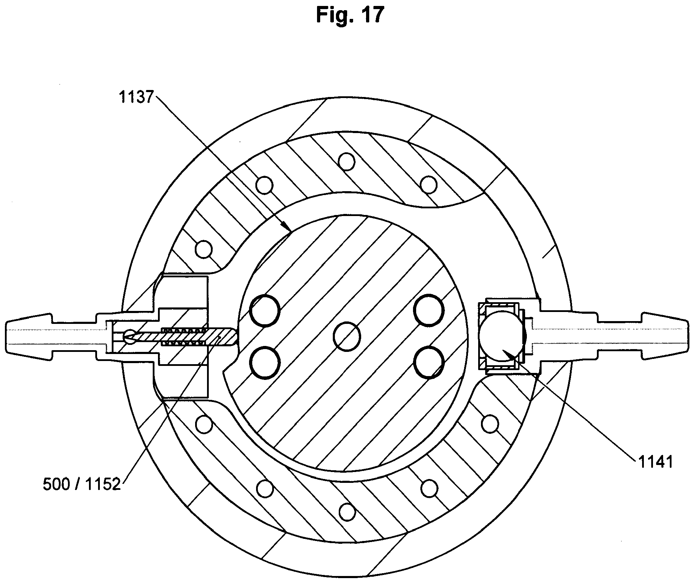

**Figur 17** zeigt einen Schnitt durch die erfindungsgemäße Vorrichtung, wobei die Mittelachse des Schließteiles 1152 in der Schnittebene liegt. Dabei verläuft die Schnittebene durch die zu dem Schließteil 1152 gehörige zweite Kurvenbahn 714. Es ist ersichtlich, dass die zu dem Schließteil 1152 gehörige zweite Kurvenbahn 714 spiralförmig verläuft, so dass zwischen zwei Extremwerten eine stufenlose Einstellung des Schließteiles 1152 möglich ist.

Vorzugsweise ist von zwei erfindungsgemäß in einem Gehäuse angeordneten Ventilen das eine Ventil am Einlass 202 des Gehäuses und das andere Ventil am Auslass 203 des Gehäuses angeordnet.

Es kommen auch andere Kombinationen von Ventilen in einem erfindungsgemäßen Gehäuse in Betracht. Dazu gehören Kombinationen von Gravitationsventilen mit anderen Ventilen als Differenzdruckventilen, wie auch Differenzdruckventile mit anderen Ventilen als Gravitationsventilen. Zu den anderen Ventilen als den Differenzdruckventilen gehören auch kurvenbahngesteuerte Ventile. Dabei wird eine Kurvenbahn an einem Schließteil entlanggeführt. Die Kurvenbahn bestimmt die Öffnungs- und Schließstellung des Schließteiles. Die Führung des Schließteiles kann formschlüssig und/oder kraftschlüssig erfolgen. Formschlüssig im Sinne der Erfindung und zugleich kraftschlüssig ist eine Nut in einem Rotor, in die das Schließteil mit einem Zapfen oder dergleichen eingreift. Die form- und kraftschlüssige Verbindung kann auch durch eine Schiene als Kurvenbahn gebildet werden, die von dem Schließteil umfasst wird. Vorzugsweise ist nur eine kraftschlüssige Verbindung zwischen der Kurvenbahn und dem Schließteil vorgesehen. Die kraftschlüssige Verbindung wird dabei durch eine Feder gebildet, mit der das Schließteil gegen die Kurvenbahn gedrückt oder gezogen wird. Noch weiter bevorzugt ist das Schließteil zumindest teilweise als Profilstab ausgebildet und in einer Führung verschiebbar gehalten oder mit einem Profilstab verbunden, der in einer Führung verschiebbar gehalten ist. Höchst bevorzugt ist ein zylindrischer Profilstab vorgesehen, der mit einem Ende unter dem Federdruck gegen die Kurvenbahn drückt und mit dem anderen Ende mit einem Ventilsitz korrespondiert. Der Ventilsitz kann ein Ring oder eine Bohrung im Gehäuse sein. Die Bohrung kann sich zum Schließteil erweitern. Die Erweiterung kann konisch sein oder eine andere Mantelfläche aufweisen.

Von Vorteil ist, wenn der Körper, also eine Nadel 1152 in dem zweiten Einsatz 1151 angeordnet ist, so dass der zweite Einsatz 1151 zusammen mit der Nadel 1152 in dem Gehäuse verbaut werden kann. In dem aus dem Gehäuse herausragenden Teil bildet der zweite Einsatz 1151 einen Anschluss für eine Drainageleitung. Am anderen Ende ragt der Einsatz in den Gehäuseinnenraum. Dort bildet der zweite Einsatz 1151 die Führung für das zylindrische Schließteil, die Nadel 1151.

Zugleich kann der zweite Einsatz 1151 einen Hohlraum bilden, in dem die Spiralfeder 1155 das zylindrische Schließteil 1152 umgibt. Für die Liquor-Führung kann in dem Bereich der oben beschriebenen Spitze des zweiten Einsatzes eine Bohrung 1156 quer zur Längsachse eingebracht sein, so dass der Liquor an der Spitze vorbei quer zur Längsrichtung des Einsatzes einströmen oder ausströmen kann. Am anderen Ende, welches der in die Gehäusebohrung abgewandt ist, gleitet das zylindrische Schließteil 1152 an einer zweiten Kurvenbahn 714. Dabei wird das Schließteil 1152 von einer umgebenden Spiralfeder 1155 gegen die zweite Kurvenbahn 714 gedrückt, so dass das Schließteil 1152 jeder Änderung der zweite Kurvenbahn 714 folgt. Für die gleitende Bohrung des Schließteiles 1152 an der zweiten Kurvenbahn ist eine Rundung des Schließteiles im Berührungsbereich von Vorteil. Die zweite Kurvenbahn 714 kann sich an der Stirnfläche und/oder an dem Mantel eines Rotors 1130 befinden. Vorzugsweise findet ein scheibenförmiger Rotor 1130 Verwendung, dessen Umfangsfläche als zweite Kurvenbahn 714 so gestaltet ist, so dass das Schließteil 1152 alle gewünschten Schließbewegungen und Öffnungsbewegungen ausführt. Das wird als Kurvenscheibe 704 bezeichnet.

Der Rotor/Kurvenscheibe ist auf einer Achse schwenkbeweglich im Ventilgehäuse gelagert. Der Rotor/Kurvenscheibe wird vorzugsweise mittels Magnete verstellt. Dazu sind einerseits in dem Rotor/Kurvenscheibe Magnete montiert und ist andererseits eine Verstelleinrichtung mit anderen Magneten vorgesehen. Die Verstelleinrichtung wird auf der Haut des Patienten über dem erfindungsgemäßen Gehäuse aufgesetzt und gedreht. Bei einer Drehung der Verstelleinrichtung folgt der Rotor/Kurvenscheibe der Magnetkraft bzw. Verstellkraft der Verstelleinrichtung. Nach jeder Verstellung wird der Rotor/Kurvenscheibe in der erreichten Stellung bis zur nächsten Verstellung arretiert. Die Arretierung erfolgt wahlweise durch Klemmung des Rotors/Kurvenscheibe. Die Klemmung kann am Umfang oder an der Stirnfläche des Rotors/Kurvenscheibe erfolgen. Zur Klemmung an dem Umfang kann das Gehäuse genutzt werden, indem ein Gehäuse mit einem nachgiebigen Deckel und nachgiebigen Seitenwänden verwendet wird, so dass die Seitenwände sich bei einem Druck auf den Gehäusedeckel nach außen aufwölben und den Rotor freigeben. Bei einer Aufhebung des Druckes formt sich das Gehäuse zurück und schließt der Rotor/Kurvenscheibe zwischen den Seitenwänden ein.

Der notwendige Druck zur Gehäuseverformung wird mit der Verstelleinrichtung erzeugt. Die Verstelleinrichtung wird deshalb zunächst zur Aufhebung der Arretierung gegen den Gehäusedeckel gedrückt, bevor das Verschwenken erfolgt.

Zur Klemmung des Rotors an der Stirnfläche ist vorzugsweise eine in axialer Richtung verstellbare Achse in dem Gehäuse vorgesehen. Die Achse steht unter dem Druck einer Feder, die den Rotor reibungsschlüssig gegen einen geringfügig nach außen gewölbten Deckel drückt. Sobald die Achse durch Druck auf den Deckel in axialer Richtung ein gewisses Maß verschoben wird, löst sich der Rotor von dem Deckel. Das gewisse Maß ist eine geringe Wölbung des Deckels nach innen. Danach kann der Rotor in der oben beschrieben Form gedreht werden. Der Druck wird mit der Verstelleinrichtung wie bei einer Arretierung am Umfang erzeugt; desgleichen die Drehung. Die Verschiebung ist im Gehäuse möglich, weil zwischen dem Ende der Achse und dem Boden des Gehäuses ein entsprechendes Spiel vorgesehen ist. Die Achse besitzt einen Bund, mit dem sie den als Kurvenscheibe ausgebildeten Rotor hintergreift. Die Feder, mit der über die Achse und deren Bund den Arretierungsdruck auf den Rotor/Kurvenscheibe aufgebracht wird, ist zwischen dem Gehäuseboden und dem Bund der Achse angeordnet. Zusätzlich kann an der dem Deckel zugewandten Seite des Rotors ein Ring montiert werden, der den Rotor bei einer nach innen gerichteten Deckelverformung zum Abheben von dem Deckel zwingt. Der Ring wird dazu an dem Rotor oder an der Achse befestigt.

Das vorstehend beschriebene Differenzdruckventil kann mit anderen Ventilen kombiniert werden. Dabei kann das Differenzdruckventil in Strömungsrichtung/Drainagerichtung vor oder hinter dem anderen Ventil angeordnet sein. In Kombination mit einem anderen Ventil, dessen Schließkörper federbelastet ist und sich entsprechend dem Liquordruck öffnet, kann das oben beschriebene Ventil genutzt werden, um die Drainagegeschwindigkeit zu dämpfen, d.h. um den Druckabfall über einen gewissen Zeitraum zu vergleichmäßigen.

Vorzugsweise finden Gravitationsventile als andere Ventile Anwendung.

Diese Gravitationsventile können oben beschriebene übliche Ventile sein.

Das Gravitationsventil öffnet in der Liegend-Lage maximal.

Das führt auch zu maximalem Druckabfall.

Mit dem erfindungsgemäßen Differenzdruckventil kann der Druckabfall in vorteilhafter Weise vergleichmäßigt werden, d.h. patientenindividuell einstellbar gemacht werden.

Wahlweise wird ein besonderes Gravitationsventil, nämlich ein schaltbares Gravitationsventil in dem Gehäuse eingesetzt. Das Gravitationsventil kann abgeschaltet und zugeschaltet werden. Dazu ist vorzugweise eine Stellvorrichtung/Schaltvorrichtung in Wirkverbindung mit dem Schließteil des Gravitationsventils vorgesehen.

Die Länge der Einbuchtungen am Rotor/Kurvenscheibe am Umfang oder an der Stirnfläche des Rotors/Kurvenscheibe ergibt sich bei einer Kombination des Gravitationsventiles mit einem zweiten, verstellbaren Ventil aus dem für das zweite Ventil gewünschten Verstellbereich.

Das vorstehende beschriebene Gravitationsventil kann auch unabhängig von der Kombination mit einem zweiten Ventil Vorteile für die Liquordrainage haben. Das heißt, das beschriebene Gravitationsventil kann auch als alleiniges Ventil zur Regelung des Liquorstroms von Vorteil sein.

Sobald die Form der einzelnen Kurvenbahnen und die Drehstellung bzw. Schwenkstellung der zugehörigen Rotor/Kurvenscheiben festliegt, kann es auch von Vorteil sein, einen gemeinsamen Rotor/Kurvenscheibe für beide Ventile im gemeinsamen Gehäuse vorzusehen. Dann sind an dem gemeinsamen Rotor/Kurvenscheibe zwei Kurvenbahnen vorgesehen, von denen die eine für das eine Ventil und die andere für das andere Ventil bestimmt ist. Die beiden Kurvenbahnen liegen dann vorzugsweise in unterschiedlichen, zu einander parallelen Ebenen. Es können aber auch beide Kurvenbahnen in einer gemeinsamen Ebene liegen. Vorzugsweise erstrecken sich die Kurvenbahnen dann auf unterschiedlichen Umfangsflächen.

Nach einem Schwenken/Drehen des Rotors/Kurvenscheiben erfolgt eine Arretierung des Rotors/Kurvenscheibe, so dass eine ungewollte Verstellung vermieden wird. Die Arretierung wird vor der Verstellung gelöst und greift nach jeder Verstellung wieder ein. Eine solche Verstellung kann mit unterschiedlichen Ausführungen erreicht werden. In einer Ausführung ist am Gehäuse und am Rotor eine Verzahnung vorgesehen. Wenn die Verzahnungen ineinandergreifen, ist der Rotor/Kurvenscheibe blockiert. Die Blockierung löst sich, indem die Verzahnungen auseinander gebracht werden.

Andere Ausführungsformen basieren darauf, dass das Gehäuse in der Arretierungsstellung reibungsschlüssig an dem Rotor/Kurvenscheibe anliegt. Zur Lösung der Arretierung wird das Gehäuse so verformt, dass es von dem Rotor/Kurvenscheibe abhebt. Die zum Lösen der Arretierung erforderliche Verformung erfolgt vorzugsweise mit der Verstellvorrichtung zum Drehen des Rotors. Zum Lösen der Arretierung wird die Verstellvorrichtung nicht nur über dem implantierten Ventil auf der Haut des Patienten aufgesetzt, sondern auch noch gegen das Ventil gedrückt, bis sich die Arretierung durch Verformung des Gehäuses gelöst hat. Nach Abheben der Verstellvorrichtung formt sich das Gehäuse automatisch zurück und greift die Arretierung.

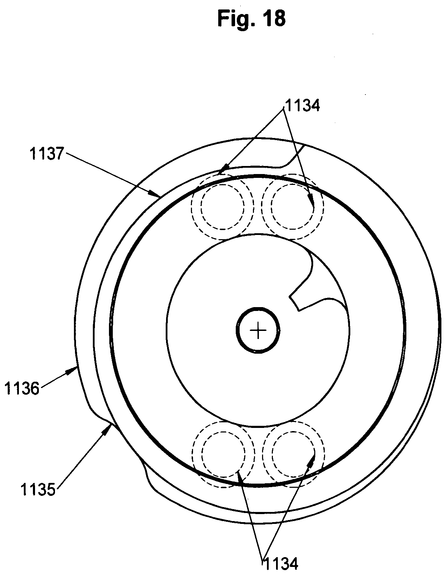

**Figur 18** zeigt eine bevorzugte Ausführungsform eines Rotors in einer Draufsicht. Zu sehen sind zwei unterschiedliche Stufen, deren jeder Außenrand eine Kurvenbahn ist. Die untere Stufe zeichnet sich durch eine erste Kurvenbahn 712, die obere Stufe durch eine zweite Kurvenbahn 714 aus. In die untere Stufe ist eine Einwölbung 1135 eingelassen, eine Auswölbung 1136 ist zu sehen.

### Bezugszeichenliste

- 100: Hydrozephalusventil

- 200: Gehäuse
- 201: Gehäuseinnenraum
- 202: Einlass
- 203: Auslass
- 204: Rohr
- 205: Rohraußendurchmesser
- 300: Durchlass
- 301: Durchlassende
- 302: Durchlassrichtung
- 303: Querschnittsfläche
- 304: Durchlassinnenfläche
- 306: Trichterförmiger Einlass
- 307: Schlauchartiger Abschnitt
- 308: Auslassbuchse

- 400: Kupplungselement
- 401: Erstes Ende
- 402: Zweites Ende
- 403: Getriebeglied
- 404: Zapfen
- 405: Linearlager
- 406: Stecker
- 407: Verschlußende
- 408: Berührabschnitt
- 409: Halsabschnitt
- 410: Kragenabschnitt
- 411: Verlängerungsabschnitt
- 412: Hülse
- 413: Hülseninnendurchmesser
- 414: Hülsenlänge
- 415: Rohrende
- 416: Zentralstecker
- 417: Rand

- 500: Körper
- 501: -
- 502: Körperachse
- 503: Körpermantelfläche
- 504: Erstes Körperende
- 505: Zweites Körperende
- 506: Keil
- 507: Keilstirnfläche
- 508: Übergangskante
- 509: Stab
- 510: Kragen

- 600: Spalt
- 601: Durchlassvolumen
- 602: Spaltlänge

- 700: Einstelleinheit
- 701: Richtung
- 702: Abstand
- 703: Berührungspunkt
- 704: Kurvenscheibe
- 705: Achse
- 706: Rotor
- 707: Magnet
- 708: Nordpol
- 709: Südpol
- 710: Anschlag
- 711: magnetisches Kupplungsglied
- 712: Erste Kurvenbahn
- 713: Erste Kurvenbahnfläche
- 714: Zweite Kurvenbahn
- 715: Zweite Kurvenbahnfläche

- 800: Federsitz
- 801: Federelement
- 802: Feder
- 803: Spiralfeder
- 804: Steg

- 900: Fluid

- 1000: Bremse
- 1050: Schwenkarm

- 1100: Ventilkombination
- 1101: Zweites Ventil
- 1102: Drainageleitung

- 1103: Druckkurve

- 1110: Boden
- 1111: Ring
- 1112: Stützscheibe
- 1113: Deckel
- 1114: Führung
- 1115: Hohlraum
- 1116: Ventilsitz
- 1117: Kanal

- 1120: Achse
- 1121: Außenkragen
- 1122: Spiralfeder

- 1130: Rotor
- 1131: Sicherungsring
- 1132: Innenkragen
- 1133: Ausnehmung
- 1134: Magnete
- 1135: Einwölbung
- 1136: Auswölbung

- 1140a: Gravitationsventil
- 1140b: Differenzdruckventil
- 1141: Kugel
- 1142: Erster Einsatz
- 1143: Anschlußtülle
- 1144: Öffnung
- 1145: Ventilsitz
- 1146: Strömungsrichtung
- 1147: Tellerfeder

- 1150: Auslass
- 1151: Zweiter Einsatz
- 1152: Körper (Nadel)
- 1153: Spitze
- 1154: Spaltdurchlass
- 1155: Spiralfeder
- 1156: Bohrung

## Patentansprüche

1. Hydrocephalusventil (100, 1100) zum Abfluss von Fluid aus Ventrikelsystemen von Patienten, das
- ein Gehäuse (200) mit Gehäuseinnenraum (201);
- und mindestens einen ersten Durchlass (300) zum Einlassen und/oder Auslassen in einer Durchlassrichtung (302) aufweist,
- und einen Körper (500, 506, 1152);
- welcher im Gehäuseinnenraum (201) angeordnet ist,
- wobei mindestens eine Einstelleinheit (700, 704, 706) vorhanden ist,
**dadurch gekennzeichnet, dass**
- der Körper (500, 506, 1152) in dem Durchlass (300) angeordnet ist und zwischen dem Körper (500, 506, 1152) und dem Durchlass (300) ein durch die Einstelleinheit (700, 704, 706) einstellbarer Spalt (600) zum Abfluss von Fluid angeordnet ist,
- wobei der Körper (500, 506, 1152) mindestens in der Durchlassrichtung (302) bewegbar ausgebildet ist,
- die Einstelleinheit (700, 704, 706) ausgebildet ist,
- mindestens eine Abflussgeschwindigkeit im Durchlass durch Bewegen des Körpers im Durchlass einzustellen, und
- die Abflussgeschwindigkeit bei einem Hydrocephalusventil (100) zwischen 1 ml pro Stunde und 1000 ml pro Stunde einstellbar macht, um durch diese Einstellung eine aus dem Abfluss resultierende Änderung des Druckes im Ventrikelsystem zu verlangsamen oder zu beschleunigen.

2. Hydrocephalusventil nach Anspruch 1, **dadurch gekennzeichnet, dass** als Körper (500, 506) mindestens ein geführter Stopfen, Keil (506), Konus, Profilstab oder eine Kugel verwendet wird, und/oder der Körper (500, 506, 1152) vorzugsweise einen Kragen (510) aufweist, wobei zwischen dem Kragen (510) und mindestens einer Teilfläche der Einstelleinheit eine Feder (802, 1155) vorgesehen ist, welche die bleibende Berührung des Körpers (500, 506, 1152) mit der Teilfläche sicherstellt.

3. Hydrocephalusventil nach einem der vorherigen Ansprüche, **dadurch gekennzeichnet, dass** der Durchlass (300) mindestens ein erster Ventileinlass (202) und/oder mindestens ein erster Ventilauslass (203) ist.

4. Hydrocephalusventil nach einem oder mehreren der vorherigen Ansprüche, **dadurch gekennzeichnet, dass** die Einstelleinheit (700) eine Kurvenscheibe (704) umfasst, die vorzugsweise eine Achse (705) aufweist, wobei die Achse (705) vor dem Durchlass (300) angeordnet ist.

5. Hydrocephalusventil nach einem oder mehreren der vorherigen Ansprüche, **dadurch gekennzeichnet, dass** die Einstelleinheit (700) mindestens einen Rotor (706) und/oder mindestens einen Magneten (707,1134) umfasst, und/oder der Rotor (706) mit dem Magneten (707,1134) und der Rotor (706) mit der Einstelleinheit (700) verbunden ausgebildet ist.

6. Hydrocephalusventil nach einem oder mehreren der vorherigen Ansprüche, **dadurch gekennzeichnet, dass** eine Bewegung der Einstelleinheit (700) eine Teilrotation und/oder eine Rotation und/oder eine Anzahl an Rotationen oder ein Schub oder ein Hub ist, und/oder die Einstelleinheit (700) die Bewegung des Körpers (500,506, 1152) entlang einer Kurvenbahn steuert, wobei die Kurvenbahn (712, 714) vorzugsweise durch die Umfangsfläche oder die Stirnfläche der Einstelleinheit (700, 704) oder des Rotors (706) gebildet wird, und/oder der Körper (500, 506, 1152) des auslaufseitigen Ventils unter Federdruck an der Kurvenbahn anliegt.

7. Hydrocephalusventil nach einem oder mehreren der vorherigen Ansprüche, **dadurch gekennzeichnet, dass** der Körper (500, 506, 1152) an seinem ersten Ende an der Kurvenbahn anliegt, und/oder der Körper (500, 506, 1152) an seinem ersten Ende mit einer Rundung an der Kurvenbahn anliegt.

8. Hydrocephalusventil nach Anspruch 2, **dadurch gekennzeichnet, dass** der Profilstab (1152) mit einer Spitze (1153) in eine Öffnung (1144) des Auslasses ragt, wobei die Spitze (1153) vorzugsweise konisch ist, noch weiter bevorzugt der Außendurchmesser des Profilstabes größer als die öffnungsweite des Auslasses ist.

9. Hydrocephalusventil nach einem oder mehreren der vorherigen Ansprüche, **dadurch gekennzeichnet, dass** der Auslass (203) eine Rohrform mit zylindrischem Innenmantel besitzt, und/oder der Auslass (203) durch einen Einsatz (1152) des Gehäuses gebildet wird und die Führung für den Körper (500, 506, 1152) durch den Einsatz des Gehäuses gebildet wird.

10. Hydrocephalusventil nach einem oder mehreren der vorherigen Ansprüche, **dadurch gekennzeichnet, dass** der Körper (500, 506, 1152) an seinem ersten Ende in dem Einsatz (1151) abstützt ist.

11. Hydrocephalusventil nach einem der vorherigen Ansprüche, **dadurch gekennzeichnet, dass** mindestens ein Hydrocephalusventil mit mindestes einem zweiten Ventil kombiniert ist, das in Strömungsrichtung des Fluids vor oder hinter dem Hydrocephalusventil angeordnet ist, und/oder das Hydrocephalusventil und das zweite Ventil in einem Strömungspfad angeordnet sind, und/oder das zweite Ventil vorzugsweise ein Differenzdruckventil (1140b) ist.

12. Hydrocephalusventil nach Anspruch 11, **dadurch gekennzeichnet, dass** das das zweite Ventil ein federbelastetes Schließteil besitzt, welches sich in Abhängigkeit von dem Liquordruck schließt und öffnet.

13. Hydrocephalusventil nach Anspruch 11 oder 12, **dadurch gekennzeichnet, dass** ein gemeinsames Gehäuse für beide Ventile vorhanden ist.

14. Hydrocephalusventil nach Anspruch 13, **dadurch gekennzeichnet, dass**
- in dem Gehäuse
- das Hydrocephalusventil dem Einlass und das zweite Ventil dem Auslass oder
- das zweite Ventil dem Einlass und das Hydrocephalusventil dem Auslass zugeordnet ist und dass in
- dem Gehäuse Kanäle von Hydrocephalusventil zum zweiten Ventil vorgesehen sind, die einen geringeren Strömungswiderstand haben als eine Verbindung beider Ventile mit einer Drainageleitung wie sich für die Zuführung von Fluid zu dem gemeinsamen Gehäuse und/oder zur Ableitung von Fluid von dem gemeinsamen Gehäuse vorgesehen sind.

15. Verfahren zum Betrieb eines Hydrocephalusventils zum Einstellen einer Abflussgeschwindigkeit nach einem der vorherigen Ansprüche, **gekennzeichnet durch** die Schritte:
- Verbringen mindestens eines Magneten in die Nähe des Hydrocephalusventils;
- Lösen einer Bremse einer Einstelleinheit des Hydrocephalusventils;
- Verdrehen der Einstelleinheit, so dass der Körper in einer vorgesehenen Position im Spalt im Hydrocephalusventil angeordnet wird.

## Claims

1. A hydrocephalus valve (100, 1100) for draining fluid from ventricular systems of patients, that has
- a casing (200) with casing interior (201);
- and at least one first passage (300) for admission and/or discharging in an passage direction (302),
- and a body (500, 506, 1152);
- which is arranged in the casing interior (201),
- wherein at least one adjusting unit (700, 704, 706) is present, **characterized in that** the body (500, 506, 1152) is arranged in the passage (300) and that an adjustable gab (600) for draining fluid is arranged between the body (500, 506, 1152) and the passage (300),
- wherein the body (500, 506, 1152) is designed to be movable at least in the passage direction (302),
- the adjusting unit (700, 704, 706) being designed
- to adjust at least a drainage rate in the passage by moving the body in the passage, and
- to make the drainage rate at a at the hydrocephalus valve (100) adjustable between 1 ml per hour and 1000 ml per hour in order, by way of this adjustment, to slow or accelerate a change, resulting from the drainage, in the pressure in the ventricular system.

2. The hydrocephalus valve as claimed in claim 1, **characterized in that** at least one guided plug, wedge (506), cone, profiled rod or a ball is used as body (500, 506), and/or **in that** the body (500, 506, 1152) preferably has a collar (510), wherein, between the collar (510) and at least one surface portion of the adjusting unit, there is provided a spring (802, 1155) which ensures permanent contact of the body (500, 506, 1152) with the surface portion.

3. The hydrocephalus valve as claimed in any of the preceding claims, **characterized in that** the passage (300) is at least one first valve inlet (202) and/or that the passage (300) is at least one first valve outlet (203).

4. The hydrocephalus valve as claimed in one or more of the preceding claims, **characterized in that** the adjusting unit (700) comprises a cam disk (704), the cam disk (704) preferably having an axis (705), wherein the axis (705) is arranged in front of the passage (300).

5. The hydrocephalus valve as claimed in one or more of the preceding claims, **characterized in that** the adjusting unit (700) comprises at least one rotor (706), and/or **in that** the adjusting unit comprises at least one magnet (707, 1134), and/or **in that** the rotor (706) is formed so as to be connected to the magnet (707, 1134) and the rotor (706) is formed so as to be connected to the adjusting unit (700).

6. The hydrocephalus valve as claimed in one or more of the preceding claims, **characterized in that** a movement of the adjusting unit (700) is a partial rotation and/or one rotation and/or a number of rotations or a sliding movement or a stroke movement, and/or **in that** the adjusting unit (700) controls the movement of the body (500, 506, 1152) along a cam track, wherein the cam track (712, 714) preferably is formed by the circumferential surface or the face surface of the adjusting unit (700, 704) or of the rotor (706), and/or **in that** the body (500, 506, 1152) of the outflow-side valve bears under spring pressure against the cam track.

7. The hydrocephalus valve as claimed in one or more of the preceding claims, **characterized in that** the body (500, 506, 1152) bears at its first end against the cam track, and/or **in that** the body (500, 506, 1152) bears at its first end with a rounded portion against the cam track.

8. The hydrocephalus valve according to claim 2, **characterized in that** the profiled rod (1152) projects with a tip (1153) into an opening (1144) of the outlet, wherein the tip (1153) is preferably conical, and even more preferably the outer diameter of the profiled rod is greater than the opening width of the outlet.

9. The hydrocephalus valve as claimed in one or more of the preceding claims, **characterized in that** the outlet (203) has a tubular form with a cylindrical inner shell, and/or **in that** the outlet (203) is formed by an insert (1152) of the casing, and the guide for the body (500, 506, 1152) is formed by the insert of the casing.

10. The hydrocephalus valve as claimed in one or more of the preceding claims, **characterized in that** the body (500, 506, 1152) is supported at its first end in the insert (1151).

11. A hydrocephalus valve, **characterized in that** at least one hydrocephalus valve is combined with at least one second valve is arranged upstream or downstream of the hydrocephalus valve in a flow direction of the fluid, and/or **in that** the hydrocephalus valve and the second valve are arranged in one flow path, and/or **in that** the second valve is a differential pressure valve (1140b).

12. The hydrocephalus valve as claimed claim 11, **characterized in that** the second valve has a spring-loaded closing part which closes and opens in a manner dependent on the fluid pressure.

13. The hydrocephalus valve as claimed in claim 11 or 12, **characterized in that** a common casing is provided for both valves.

14. The hydrocephalus valve as claimed in claim 13, **characterized in that**,
- in the casing,
- the hydrocephalus valve is assigned to the inlet and the second valve is assigned to the outlet,
or
the second valve is assigned to the inlet and the hydrocephalus valve is assigned to the outlet, and **in that**, in
- the casing, channels are provided from the hydrocephalus valve to the second valve, which channels have a lower flow resistance than a connection of the two valves to a drainage line as are provided for the feed of fluid to the common casing and/or for the drainage of fluid from the common casing.

15. A method for operating a hydrocephalus valve for adjusting a drainage rate according to one of the preceding claims, **characterized by** the steps:
- moving at least one magnet into the vicinity of the hydrocephalus valve;
- releasing a brake of an adjusting unit of the hydrocephalus valve;
- rotating the adjusting unit such that the body is arranged in an intended position in the gap in the hydrocephalus valve.

## Revendications

1. Valve d'hydrocéphalie (100, 1100) pour l'évacuation du fluide des systèmes ventriculaires des patients, le
- un boîtier (200) avec un espace intérieur de boîtier (201) ;
- et au moins un premier passage (300) pour l'entrée et/ou la sortie dans une direction de passage (302),
- et un corps (500, 506, 1152) ;
- qui est disposé dans l'espace intérieur du boîtier (201),
- dans lequel au moins une unité de réglage (700, 704, 706) est présente,
**caractérisé en ce que**
- le corps (500, 506, 1152) est disposé dans le passage (300) et un espace (600) réglable par l'unité de réglage (700, 704, 706) est disposé entre le corps (500, 506, 1152) et le passage (300) pour l'évacuation du fluide,
- dans lequel le corps (500, 506, 1152) est conçu pour être mobile au moins dans la direction de passage (302),
- l'unité de réglage (700, 704, 706) est formée,
- d'ajuster au moins une vitesse d'écoulement dans le passage en déplaçant le corps dans le passage, et
- permet de régler la vitesse d'écoulement dans une valve d'hydrocéphalie (100) entre 1 ml par heure et 1000 ml par heure, afin de ralentir ou d'accélérer par ce réglage une modification de la pression dans le système ventriculaire résultant de l'écoulement.

2. Valve d'hydrocéphalie selon la revendication 1, **caractérisée en ce que** l'on utilise comme corps (500, 506) au moins un bouchon guidé, une cale (506), un cône, une barre profilée ou une bille, et/ou le corps (500, 506, 1152) présente de préférence une collerette (510), un ressort (802, 1155) étant prévu entre la collerette (510) et au moins une surface partielle de l'unité de réglage, lequel assure le contact permanent du corps (500, 506, 1152) avec la surface partielle.

3. Valve d'hydrocéphalie selon l'une quelconque des revendications précédentes, **caractérisée en ce que** le passage (300) est au moins une première entrée de valve (202) et/ou au moins une première sortie de valve (203).

4. Valve d'hydrocéphalie selon l'une ou plusieurs des revendications précédentes, **caractérisée en ce que** l'unité de réglage (700) comprend une came (704) ayant de préférence un axe (705), l'axe (705) étant situé en amont du passage (300).

5. Valve d'hydrocéphalie selon une ou plusieurs des revendications précédentes, **caractérisée en ce que** l'unité de réglage (700) comprend au moins un rotor (706) et/ou au moins un aimant (707, 1134), et/ou le rotor (706) est formé avec l'aimant (707, 1134) et le rotor (706) est formé avec l'unité de réglage (700).

6. Valve d'hydrocéphalie selon une ou plusieurs des revendications précédentes, **caractérisée en ce qu'**un mouvement de l'unité de réglage (700) est une rotation partielle et/ou une rotation et/ou un nombre de rotations ou une poussée ou une course, et/ou l'unité de réglage (700) contrôle le mouvement du corps (500, 506, 1152) le long d'une trajectoire courbe, la trajectoire courbe (712, 714) étant de préférence formée par la surface périphérique ou la surface frontale de l'unité de réglage (700, 704) ou du rotor (706), et/ou le corps (500, 506, 1152) de la valve côté sortie s'appuie sur la trajectoire courbe sous la pression d'un ressort.

7. Valve d'hydrocéphalie selon l'une ou plusieurs des revendications précédentes, **caractérisée en ce que** le corps (500, 506, 1152) est en appui à sa première extrémité contre le chemin de came, et/ou le corps (500, 506, 1152) est en appui à sa première extrémité contre le chemin de came avec un arrondi.

8. Valve d'hydrocéphalie selon la revendication 2, **caractérisée en ce que** la tige profilée (1152) fait saillie par une pointe (1153) dans une ouverture (1144) de l'orifice de sortie, la pointe (1153) étant de préférence conique, de préférence encore le diamètre extérieur de la tige profilée est supérieur à la largeur d'ouverture de l'orifice de sortie.

9. Valve d'hydrocéphalie selon l'une ou plusieurs des revendications précédentes, **caractérisée en ce que** la sortie (203) a une forme tubulaire avec une enveloppe intérieure cylindrique, et/ou la sortie (203) est formée par un insert (1152) du corps et le guide pour le corps (500, 506, 1152) est formé par l'insert du corps.

10. Valve d'hydrocéphalie selon l'une ou plusieurs des revendications précédentes, **caractérisée en ce que** le corps (500, 506, 1152) est supporté à sa première extrémité dans l'insert (1151).

11. Valve d'hydrocéphalie selon l'une quelconque des revendications précédentes, **caractérisée en ce qu'**au moins une valve d'hydrocéphalie est combinée avec au moins une deuxième valve qui est disposée en amont ou en aval de la valve d'hydrocéphalie dans le sens d'écoulement du fluide, et/ou la valve d'hydrocéphalie et la deuxième valve sont disposées dans un trajet d'écoulement, et/ou la deuxième vanne est de préférence une valve à pression différentielle (1140b).

12. Valve d'hydrocéphalie selon la revendication 11, **caractérisée en ce que** la deuxième valve possède un élément de fermeture chargé par ressort qui se ferme et s'ouvre en fonction de la pression du liquide céphalorachidien.

13. Valve d'hydrocéphalie selon la revendication 11 ou 12, **caractérisée en ce qu'**il existe un boîtier commun pour les deux valves.

14. Valve d'hydrocéphalie selon la revendication 13, **caractérisée en ce que**
- dans le boîtier
- la valve d'hydrocéphalie à l'entrée et la deuxième valve à la sortie, ou
- la deuxième valve est associée à l'entrée et la valve d'hydrocéphalie à la sortie, et **en ce que**, dans
- le boîtier comporte des canaux allant de la valve hydrocéphale à la seconde valve, qui ont une résistance à l'écoulement inférieure à celle d'une connexion des deux soupapes à un conduit de drainage tel que prévu pour l'alimentation en fluide du boîtier commun et/ou pour l'évacuation de fluide du boîtier commun.

15. Procédé de fonctionnement d'une valve d'hydrocéphalie pour ajuster un débit d'écoulement selon l'une quelconque des revendications précédentes, **caractérisé par** les étapes consistant à :
- Placer au moins un aimant à proximité de la valve hydrocéphalique ;
- Desserrer un frein d'une unité de réglage de la valve hydrocéphale ;
- faire tourner l'unité de réglage de manière à ce que le corps soit placé dans une position prévue dans la fente dans la valve hydrocéphale.
